(19)

Europäisches
Patentamt
European
Patent Office
Office européen
des brevets

(11)     **EP 3 404 113 A1**

(12)     **EUROPEAN PATENT APPLICATION**

(43) Date of publication:
**21.11.2018 Bulletin 2018/47**

(51) Int Cl.:
***C12Q 1/68*** *(2018.01)*

(21) Application number: **17382287.5**

(22) Date of filing: **19.05.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Universidad del Pais Vasco
48940 Leioa Vizcaya (ES)**

(72) Inventors:
• **RODRÍGUEZ LARREA, David
E-48940 Leioa - Vizcaya (ES)**
• **CELAYA GARAVILLA, Garbiñe
E-48940 Leioa - Vizcaya (ES)**

(74) Representative: **ABG Intellectual Property, S.L.
Avenida de Burgos, 16D
Edificio Euromor
28036 Madrid (ES)**

(54)     **METHOD FOR DETECTING PROTEIN-DNA INTERACTION**

(57)     The present invention relates to a method for detecting the interaction between a protein and a DNA target sequence based on measuring the ionic current trough an alpha hemolysin nanopore and to a method for identifying a compound capable of modulating the interaction between a protein and a DNA target sequence. In addition, the invention relates to a kit comprising at least one protein, at least one DNA molecule, said DNA molecule comprising region and a dsDNA region, wherein the dsDNA region contains the DNA target sequence, and a planar lipid bilayer containing at least one alpha hemolysin nanopore, and to the use of said kit for detecting protein-DNA interaction and/or for identifying a compound capable of modulating protein-DNA interaction.

EP 3 404 113 A1

**Description**

**Technical Field of Invention**

The present invention relates to a method for detecting protein-DNA interaction.

**Background of the invention**

The genetic information encoded in DNA is expressed and replicated under the tight control exerted by proteins. To do so, proteins recognize specific sequences on the genome. Following recognition other proteins are recruited and/or changes in DNA structure are induced, which produces an outcome: gene expression/repression or DNA replication. Mechanistically, this apparent simple scheme may be complicated because, depending on post-translational modifications,[1] chaperone action[2,3] or ligand binding,[4] the same protein can act either as an activator or a repressor. In addition, the same protein can bind different target sequences, which suggests different modes of action depending on the affinity for each site.[5,6] Finally, the concomitant binding of different transcription factors to adjacent target sequences affects DNA structure and ultimately gene expression.[5,6] Given their roles, transcription factors and plasmid replication factors are ideal targets for cancer treatment and antimicrobial agents, yet these targets are rarely exploited.

Due to their biological significance and complexity, protein-DNA interactions are the subject of intense research and several methods have been developed for their study. Electrophoretic mobility shift assays can provide at best a good estimate of $K_d$ values. Isothermal titration calorimetry renders more information on the interaction but requires large amounts of precious sample, and when large amounts are used the measurement of tight affinities is complicated. Other methods such as surface plasmon resonance allow to measure the kinetics of the interaction but requires surface immobilization of one of the macromolecules. Chromatin immunoprecipitation is gaining use because it offers a high-throughput evaluation of protein-DNA interactions *in vivo*, but it is laborious, requires the use of antibodies targeting the proteins of interest and it does not provide $K_d$ values.[5] Fluorescence methods instead can measure a wide range of affinities[7] and in addition, without the aid of rapid mixing methods, can resolve the kinetics of such interactions.[8] Further, recent fluorescence single-molecule experiments show that protein-DNA complexes manifest diverse states not observable with ensemble measurements.[9] This increase in mechanistic insight is at the cost of labelling, immobilization and may require costly and complex instrumentation.[8,10]

Therefore, there is a need in art for developing new methods for detecting protein-DNA interaction.

**Summary of the Invention**

In a first aspect, the invention relates to a method for detecting the interaction between a protein and a DNA target sequence which comprises:

a) putting into contact the protein with a DNA molecule, said DNA molecule comprising a ssDNA region and a dsDNA region, wherein the dsDNA region contains the DNA target sequence, with a planar lipid bilayer containing at least one alpha hemolysin nanopore under conditions allowing the interaction between the protein, the DNA molecule and the planar lipid bilayer,
b) applying a positive electric potential across the lipid bilayer which is capable of electrophoretically driving the ssDNA through the alpha-hemolysin nanopore within the lipid bilayer, and
c) measuring the ionic current through the alpha-hemolysin nanopore,

wherein if an initial level in the ionic current signal having higher conductance is detected, is indicative that there is protein-DNA interaction.

In a second aspect, the invention relates to a method for identifying a compound capable of modulating the interaction between a protein and a DNA target sequence:

a) putting into contact the compound with the protein, a DNA molecule, said DNA molecule comprising a ssDNA region and a dsDNA region, wherein the dsDNA region contains the DNA target sequence and with a planar lipid bilayer containing at least one alpha hemolysin nanopore, under conditions allowing the interaction between the compound, the protein, the DNA molecule and the planar lipid bilayer,
b) applying a positive electric potential across the lipid bilayer which is capable of electrophoretically driving the ssDNA through the alfa hemolysin nanopore within the lipid bilayer, and
c) measuring the ionic current through the alpha-hemolysin nanopore,

wherein if an initial level in the ionic current signal having higher conductance is detected in comparison with the signal in the absence of said compound, is indicative the compound is an enhancer of the protein-DNA interaction, and wherein if an initial level in the ionic current signal having higher conductance is not detected in comparison with the signal in the absence of said compound, is indicative the compound is an inhibitor of the protein-DNA interaction.

In a third aspect, the invention relates to a kit comprising at least one protein, at least one DNA molecule, said DNA molecule comprising a ssDNA region and a dsDNA region, wherein the dsDNA region contains the DNA target sequence; and a planar lipid bilayer containing at least one alpha hemolysin nanopore.

In a fourth aspect, the invention relates to the use of the kit of the invention for detecting protein-DNA interaction and/or for identifying a compound capable of modulating protein-DNA interaction.

**Brief Description of the Figures**

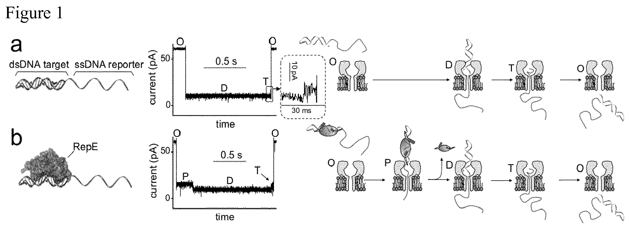

Figure 1 Experimental approach to the detection of free and protein-bound DNA molecules. (a) On the left, a molecular representation of the oligonucleotide design, containing a protein-binding dsDNA sequence followed by a ssDNA reporter. When subjected to electrophoresis through the $\alpha$-HL pore, the process gives a characteristic signal with 3 ionic current levels that correspond to different states in the unzipping and translocation process. O, the open pore state; D, the threading of the ssDNA reporter; and T, a translocation intermediate. The corresponding molecular model is depicted on the right. (b) Molecular representation of the oligonucleotide reporter bound to monomeric RepE. The characteristic ionic current signal has now an initial extra level P, which reports on the protein bound state. Following protein release both the ionic current signal and the molecular mechanism proceed as in (a).

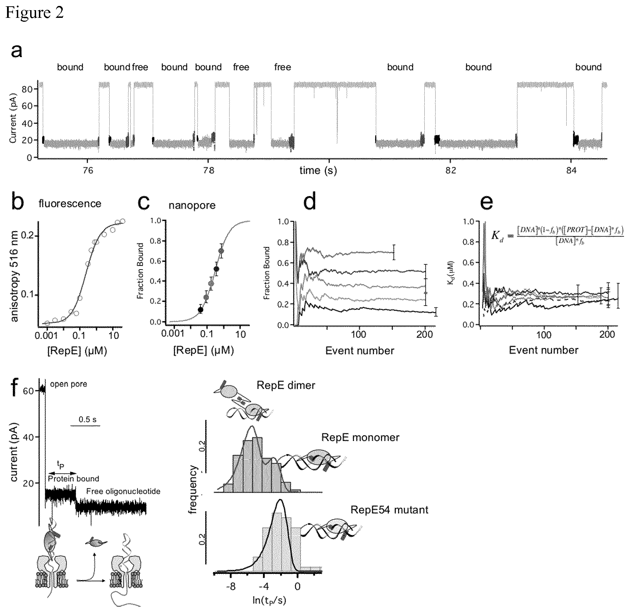

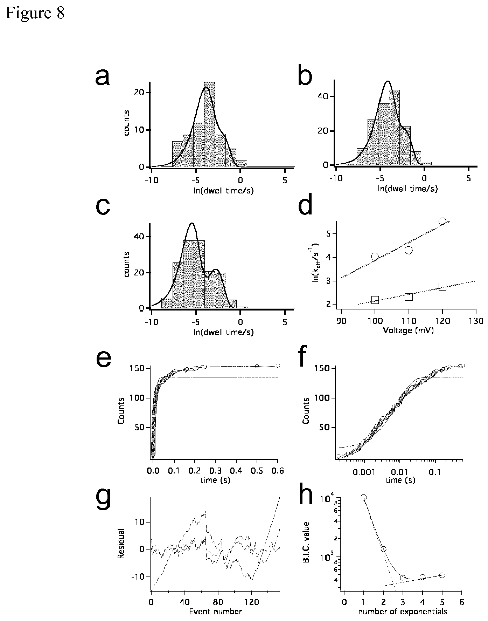

Figure 2. Determination of equilibrium and kinetic binding constants. (a) Representative ionic current recording of a mixture of free and RepE-bound DNA reporters in 150 mM KCl, 5 mM MgCl2, 10 mM HEPES, pH 7.2, 49 nM DNA and 670 nM RepE at +120 mV. Level P is depicted in black, level D in blue and level T in red while the rest of the experimental signal is in grey. (b) Binding curve of RepE-DNA measured with fluorescence anisotropy. The line represents the best fit to a one-site reversible binding model ([DNA] = 0.02 $\mu$M; Kd = 0.23 $\pm$ 0.03 $\mu$M; 1$\sigma$ error in the parameter estimation of the fit). (c) Binding curve of RepE-DNA determined from the fraction of bound events detected with the nanopore. The line represents the best fit to a one-site reversible binding model ([DNA] = 0.04 $\mu$M; Kd = 0.27 $\pm$ 0.01; 1$\sigma$ error in the parameter estimation of the fit), error bars represent the 95 % C.I. (d) Fraction of protein-bound DNA molecules found on the experiments shown in (c), with the same colour code. The number of signals that exhibited level P was divided by the number of signals counted (event number). Note that the fraction bound is estimated along the experiment. Error bars represent the 95 % C.I. in the estimation and for clarity are only reported at the end of the experiment. (e) Kd value calculated from the fraction of protein-bound DNA molecules shown in (d). The inset is the Kd formula with fd representing the fraction of DNA molecules that had a protein bound, [DNA] the concentration of DNA and [PROT] the concentration of protein. Protein:DNA ratio does not affect the estimation nor does the voltage in the +100 to +130 mV range (in solid blue the estimation at +120 mV; in dashed blue the estimation at +130 mV; and in dotted blue the estimation at +100 mV), error bars represent the 95 % C.I.. (f) Analysis of the dwell time in level P (protein-bound state). On the left, experimental signal and molecular model showing the step in the ionic current signal that is analysed. On the right the dwell time distribution of the WT RepE-DNA complex (upper panel, n = 3) and of the monomeric mutant RepE54-DNA complex (lower panel, n = 3). The lines represent the best fits to a double exponential distribution (for WT, see Figure 8) or to a single exponential distribution (for RepE54).

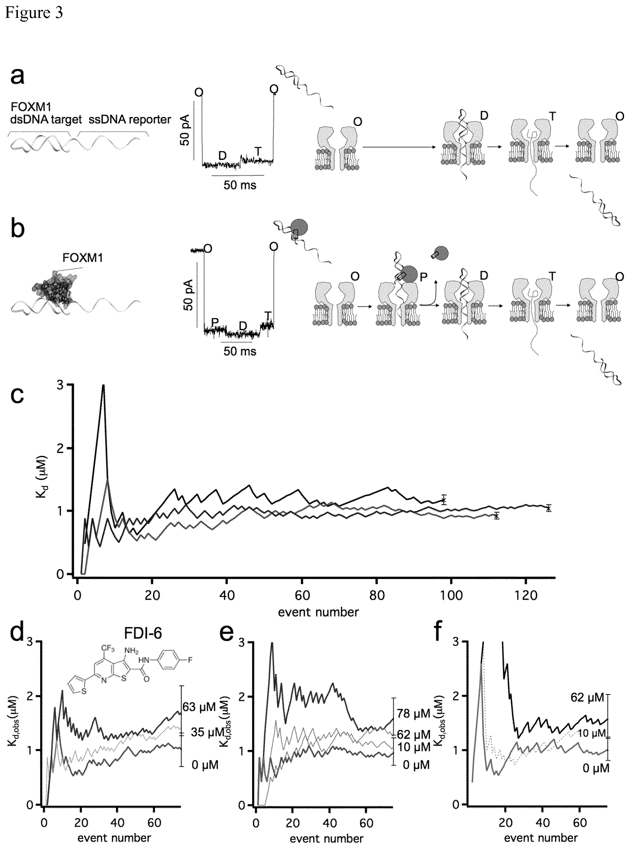

Figure 3. Single-molecule measurement of the inhibition of FOXM1 binding. (a) Molecular representation of the oligonucleotide designed to study FOXM1 binding to its target sequence, and the characteristic ionic signal it produces. The different ionic current levels are assigned to the open pore state (O), threading of the oligonucleotide (D), and an intermediate in the unzipping and translocation process (T) (right). (b) Molecular representation of FOXM1 bound to the DNA and the characteristic signal produced when the complex is analysed by a nanopore. The additional ionic level (P) represents the protein-bound state. Once FOXM1 is released, the process proceeds as in (a). (c) Real time $K_d$ estimation of the FOXM1-DNA interaction derived from the fraction of bound and unbound events (three independent experiments shown in red, blue and black, with [DNA] = 0.07, 0.106 and 0.106 $\mu$M and [FOXM1] = 0.44, 0.93 and 0.93 $\mu$M respectively). Error bars represent 1$\sigma$ C.I. (d) Observed $K_d$ when the solution used for the $K_d$ estimation in (c, red) was titrated with increasing concentrations of FDI-6. Error bars represent 1$\sigma$ C.I. (for clarity only represented in the upper and lower curves). The structure of the inhibitor FDI-6 is displayed atop the curves. (e) Observed $K_d$ when the solution used for the $K_d$ estimation in (c, blue) was titrated with increasing concentrations of FDI-6. Error bars represent 1$\sigma$ C.I. (for clarity only represented in the upper and lower curves). (f) Observed $K_d$ when the solution used for the $K_d$ estimation in (c, green) was titrated with increasing concentrations of FDI-6. Error bars represent 1σ C.I. (for clarity only represented in the upper and lower curves). In all our experiments increasing concentrations of FDI-6 produced an increase in the observed $K_d$ (n = 24).

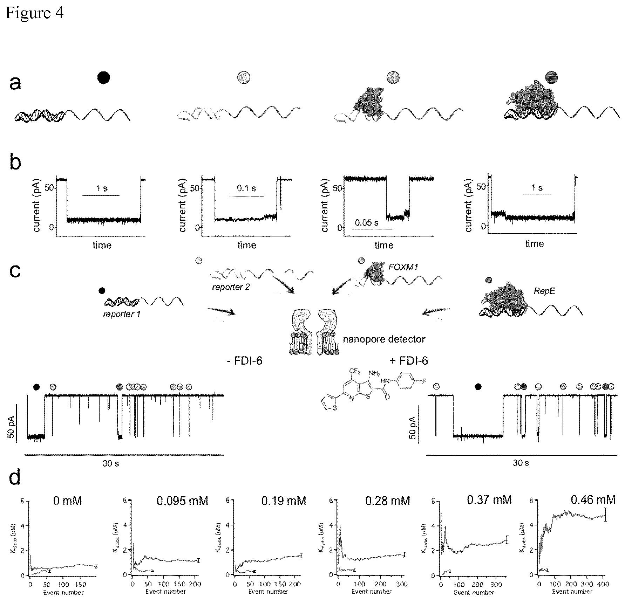

Figure 4. Multiplexed single-molecule measurements of binding inhibition. (a) Molecular representation of the four molecular species present in solution. Note that ssDNA regions of Reporters 1 and 2 differ being, respectively: poly$(dA)_{11}$-$(dC)_{20}$ and poly$(dC)_{10}$-$(dA)_{20}$. (b) Characteristic experimental ionic current signals associated with the species depicted above them. (c) Representation of the experimental set-up. Four molecular species are present in solution. Molecules are analysed one by one by a single nanopore detector. Analyses of the ionic current signals provide the identity of each event. The experiment is performed both in the absence and presence of an inhibitor (FDI-6). (d) $K_d$ estimation of RepE-DNA (green, 0.87 μM RepE and 0.047 μM of DNA) and FOXM1-DNA (magenta, 1.75 μM FOXM1 and 0.097 μM of DNA) interactions in the absence and presence of increasing concentrations of FDI-6 (error bars represent 1σ C.I.). While FOXM1-DNA interactions were inhibited by FDI-6 (noted as an increase in the observed $K_d$), in none of our measurements was the RepE-DNA pair affected (n = 4).

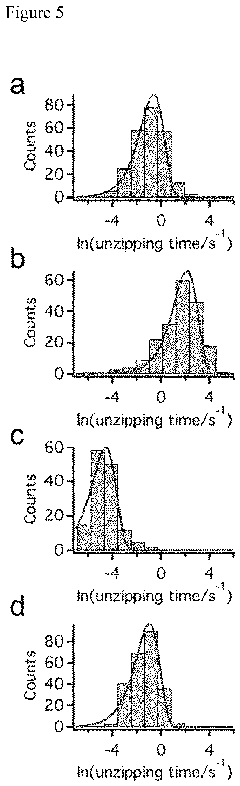

Figure 5. Kinetics of DNA unzipping. (a) Histogram of unzipping times histogram for a hairpin containing the binding sequence of RepE at +120 mV. The line represents the best fit to a single exponential distribution with $k_{unz,+120mV}$ = 1.8 [3 - 0.6] s$^{-1}$, 95% CI, n = 7. (b) Histogram of unzipping times for the same hairpin at +100 mV with the line representing the best fit to a single exponential distribution with $k_{unz,+100mV}$ = 0.12 [0.2 - 0.04] s$^{-1}$, 95% CI, n = 7. (c) Histogram of unzipping times for a hairpin containing base-pair mismatch within the RepE binding sequence at +120 mV. The line represents the best fit to a single exponential distribution with $k_{unz,+120mV}$ = 96 [162 - 30] s$^{-1}$, 95% CI, n = 3. (d) Histogram of unzipping times for the hairpin containing the binding sequence of RepE at +120 mV in complex with the protein. The line represents the best fit to a single exponential distribution with $k_{unz,+120mV}$ = 2.6 [4.4 - 0.8] s$^{-1}$, 95% CI, n = 7.

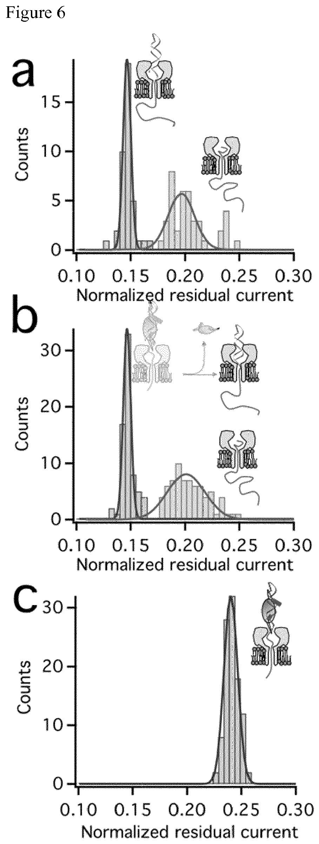

Figure 6. Normalized residual current of levels D, T and P at +120 mV. (a) Histogram of the normalized residual current for level D in red (before hairpin unzipping) and for level T in green (translocating DNA) for free DNA molecules. (b) Histogram of the normalized residual current for level D in red and level T in green for protein-bound DNA molecules. (c) Histogram of the normalized residual current for level P (protein bound state). Data was collected on the same pore to avoid pore to pore variation.

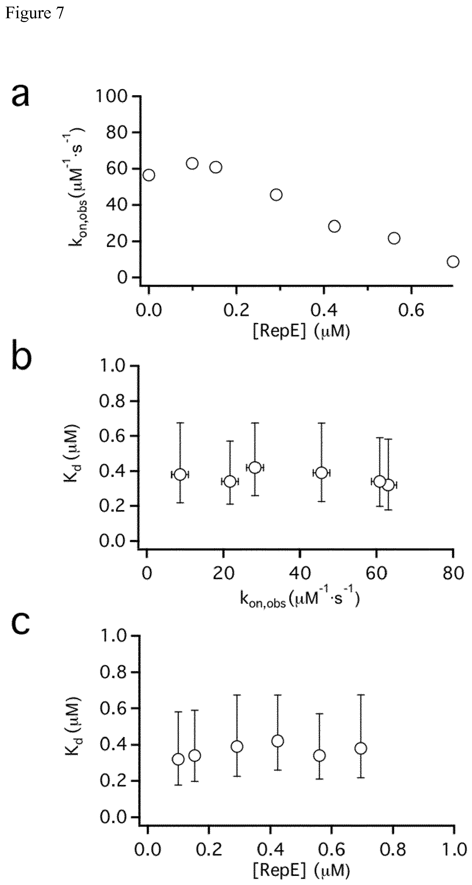

Figure 7. $K_d$ independence on the frequency of events. (a) Frequency of events ($k_{on,obs}$) as a function of RepE concentration. There is a significant decrease in the number of observed events at high RepE concentration. (b) $K_d$ value obtained as a function of the observed frequency of events. The $K_d$ value depends on the proportion of free and protein-bound molecules detected by the nanopore. This proportion is not affected by the frequency of events (*i.e.* the decrease in frequency affects both the free and bound species). (c) $K_d$ dependence on protein concentration. While the frequency of events decreases at high protein concentration, the $K_d$ value remains the same.

Figure 8. Voltage dependence of RepE detachment. (a) Histogram of the dwell time at level P (i.e. protein-bound state) at +120 mV. The line represents the best fit to a double exponential distribution with $k_{off1}$ = 256 [423 - 88.7] s$^{-1}$ and $k_{off2}$ = 17 [28 - 6] s$^{-1}$. (b) Histogram of the dwell time at level P at +110 mV. The line represents the best fit to a double exponential distribution with $k_{off1}$ = 74 [129 - 19] s$^{-1}$ and $k_{off2}$ = 10 [17 - 3] s$^{-1}$. (c) Histogram of the dwell time at level P at +100 mV. The line represents the best fit to a double exponential distribution with $k_{off1}$ = 57 [99 - 15] s$^{-1}$ and $k_{off2}$ = 9 [16 - 2] s$^{-1}$. (d) Dependence of the $k_{off}$ with voltage, note the natural logarithm scale on the left axis. In red, the dependence of $k_{off1}$ (attributed to the dimer) and in blue the dependence of $k_{off2}$ (attributed to the monomer). 95% confidence interval in the parameter estimation is provided in brackets. Data was collected in 3 independent experiments. The $k_{off}$ values at 0 mV are 0.5 [1.3 - 0.2] s$^{-1}$ and 0.03 [0.6 - 0.001] s$^{-1}$ for the monomer and the dimer respectively. 68% CI derived from the parameter estimation in the linear fit shown. (e) Cumulative histogram of RepE detachment at +120 mV. The blue line is the fit to a single exponential, the red line is the fit to a double exponential and the green line the fit to a triple exponential. (f) Same as in (e) but in logarithmic scale to allow evaluation of the quality of the fits in all the time range. (g) Residuals of the fits presented in (e). (h) Bayesian Information Criteria values (BIC values) as function of the number of exponentials used to fit the data (note that each exponential adds 2 parameters). While the lowest value is for three exponentials, the projections of the arms suggest that the lowest value would occur between two and three exponentials. We choose two exponentials because it is in agreement with the coexistence of monomers and dimers.

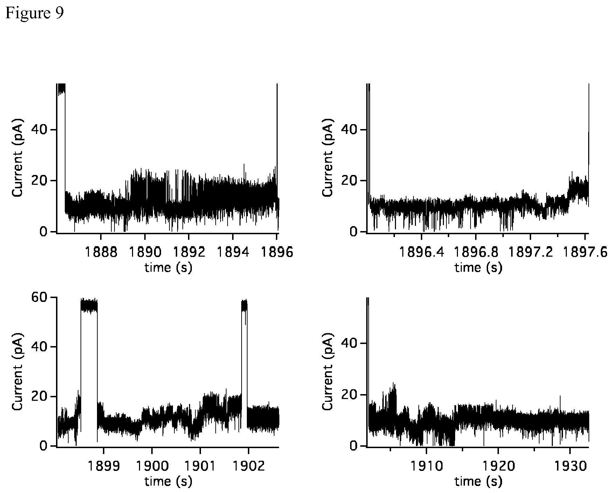

Figure 9. Effect of a DNA interacting compound. Representative ionic current traces obtained upon addition of 2.5 mM doxorubicin to a solution of RepE and its DNA-hairpin reporter. Under this conditions no characteristic signal for DNA unzipping and translocation was detected. The events instead are characterized by their length and lack of pattern.

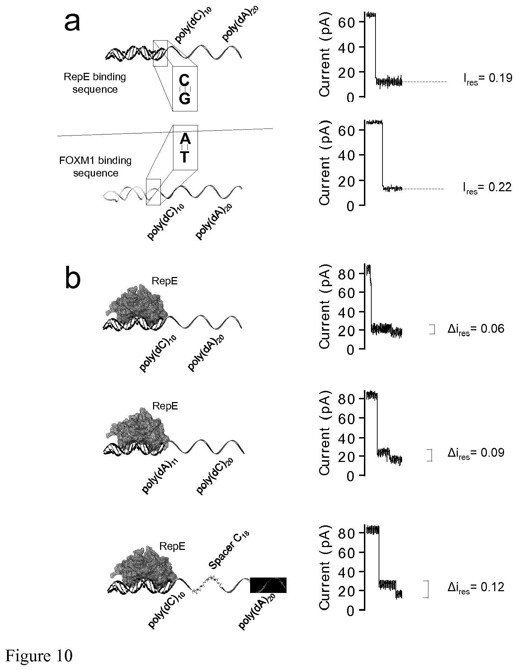

Figure 10. Residual current of different oligonucleotide sequences. (a) Molecular model of the binding sequences of RepE and FOXM1 followed by the same ssDNA sequence. The residual current seems to depend on the first base pair of the dsDNA, an interpretation in agreement with our molecular model where the dsDNA is found at the constriction of the transmembrane region of the pore. Measurements were made at +100 mV (b) Molecular model of RepE bound to oligonucleotides of different sequences measured at +120 mV. The residual current at level P can be modulated by the sequence found in the ssDNA, from base 10 to the 5' end.

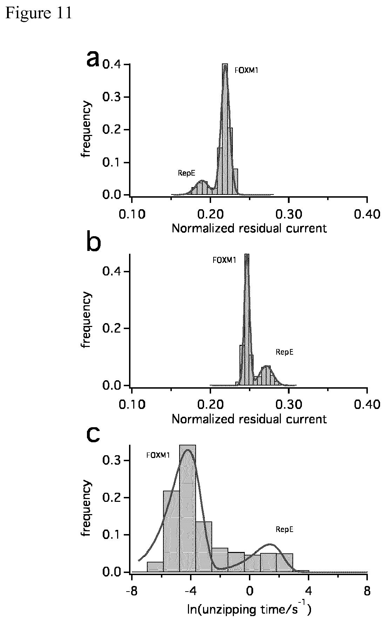

Figure 11. Parameters of the signal used for event classification. An initial solution of 1.75 $\mu$M FOXM1 and 0.097 $\mu$M Reporter 2 was analysed with a nanopore at +100 mV. Next 0.87 $\mu$M RepE and 0.047 $\mu$M Reporter 1 were added and examined with the nanopore at 0, 0.098 mM, 0.188 mM, 0.28 mM, 0.37 mM and 0.46 mM FDI-6. The residual currents at levels P, D and the dwell time at level D were examined at each FDI-6 concentration and used to make the following histograms: (a) Histogram of the normalized residual current at level D. The solid line represents the best fit to a normal distribution with two populations with $I_{D,RepE} = 0.189 \pm 0.0083$ and $I_{D,FOXM1} = 0.219 \pm 0.0053$ (mean $\pm$ S.D.), Chi-square = 0.003324. (b) Histogram of the normalized residual current at level P. The solid line represents the best fit to a normal distribution with two populations with $I_{P,RepE} = 0.271 \pm 0.00838$ and $I_{P,FOXM1} = 0.246 \pm 0.003385$ (mean $\pm$ S.D.), Chi-square = 0.00054. (c) Histogram of the unzipping times (i.e. dwell time at level D). The solid line represents the best fit to a double exponential distribution with $t_{D,RepE} = 0.25$ s$^{-1}$ and $t_{D,FOXM1} = 66$ s$^{-1}$, Chi-square = 0.009177. A total of 2081 events were used to construct histograms (a) and (c), and 1043 for (b). This experiment was repeated 4 further times with similar results.

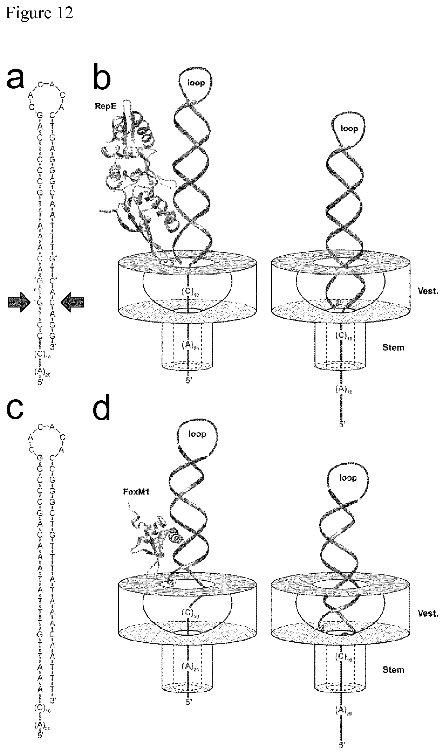

Figure 12. Molecular models explaining the approach. (a) Oligonucleotide used for the detection of RepE binding (RepE Direct). In red the consensus binding sequence. With an asterisk the nucleotides that have bases interacting with the protein. The arrows highlight the bases where a mismatch was introduced (the G was changed by a C in the Mismatched oligonucleotide) or the binding sequence altered (the G-C base pair was changed to C-G). (b) The DNA acquires two different configurations in the hemolysin pore depending on whether RepE is bound or not. The protein bound state in the left (pdb entry code 2Z9O, only one of the monomers has been represented for simplicity) does not allow the hairpin to enter the vestibule of the pore and the narrower part of the pore (stem) is filled with adenines. When the protein is not bound, the hairpin can penetrate through the vestibule and makes close contact with the narrower part of the pore (right). (c) Oligonucleotide used for the detection of FOXM1 binding. In red the consensus binding sequence. (d) Structure of FOXM1 bound to the DNA (pdb entry code 3G73, 2 nucleotides at the 3' end were not present in the structure and had to be added). As in (b), when the protein is bound to the DNA the hairpin cannot reach the vestibule of the pore (left). Instead, when the protein is detached, the hairpin enters the vestibule and makes close contact with the narrower part of the pore (right).

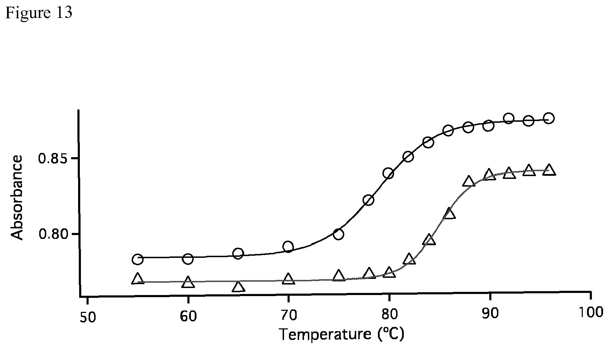

Figure 13. Thermal unzipping of the oligonucleotides used for RepE and FOXM1 binding. The circles are the absorbance at 260 nm of the FOXM1 oligonucleotide and the triangles the absorbance at 260 nm of the RepE oligonucleotide. The black and red lines are sigmoidal fittings carried out to obtain the melting temperature (Tm, the temperature at which half of the oligonucleotide sample dehybridized), giving $T_{M,FOXM1} = 79$ °C and $T_{m,RepE} = 85$ °C.

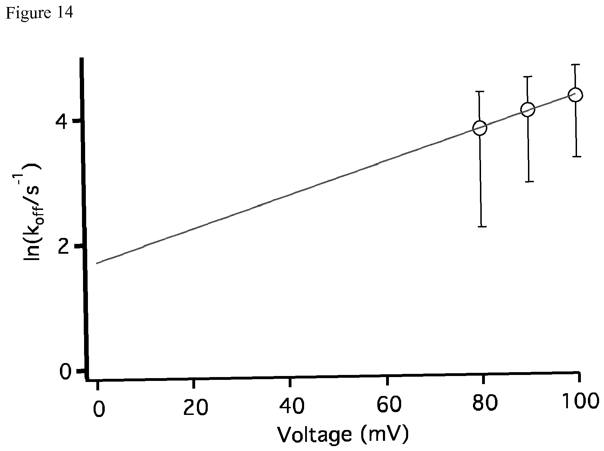

Figure 14. Kinetics of FOXM1 detachment as a function of Voltage. The linear fit gives an extrapolated value for the $k_{off}$ at 0 mV of 5.5 [6.2 - 4.8] s$^{-1}$, 68% CI derived from the estimation of the fit parameter.

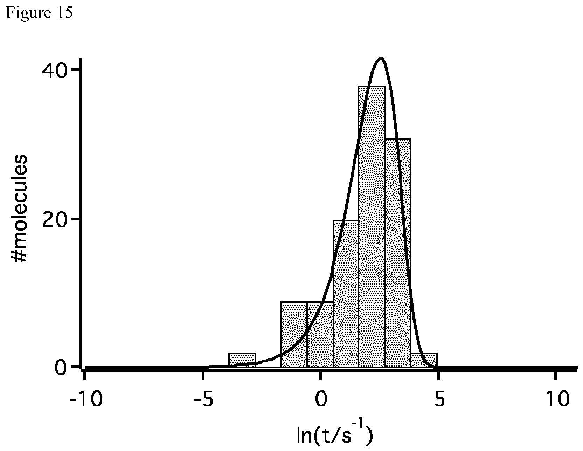

Figure 15. Histogram of the unzipping times of the RepE (INVERSE*) oligonucleotide at +100 mV with the first base-pair C-G formed. The black line is the exponential fit with $k_{unz,100mV} = 0.08$ [0.13 - 0.03] s$^{-1}$, 95% CI.

## Detailed Description of the Invention

The inventors developed an approach that allows the biophysical characterization of protein-DNA interactions and the evaluation of small compound activity in a multiplexed platform. To detect protein-DNA interactions they used a ssDNA with a ds hairpin containing the binding sequence of a target protein. When the DNA is driven through the $\alpha$-hemolysin nanopore ($\alpha$-HL) the dsDNA remains outside the pore if there is a protein bound to it, and enters the vestibule of the pore when there is no protein bound (Figure 1). The two different states produce different current profiles that allow the ready determination of the equilibrium and kinetic constants of the interaction ($K_d$ and $k_{off}$). Using this approach they show that different conformations of a protein bound to the DNA can be detected. The approach can also detect the inhibitory effect of small molecules on the DNA-protein interaction, and provide information on whether the compound binds to the DNA or the protein.

Method for detecting protein-DNA interaction

In a first aspect, the invention relates to a method for detecting the interaction between a protein and a DNA target sequence which comprises:

a) putting into contact the protein with a DNA molecule, said DNA molecule comprising a ssDNA region and a dsDNA region, wherein the dsDNA region contains the DNA target sequence, with a planar lipid bilayer containing at least one alpha hemolysin nanopore under conditions allowing the interaction between the protein, the DNA molecule and the planar lipid bilayer,
b) applying a positive electric potential across the lipid bilayer which is capable of electrophoretically driving the ssDNA through the alpha-hemolysin nanopore within the lipid bilayer, and

c) measuring the ionic current through the alpha-hemolysin nanopore,

wherein if an initial level in the ionic current signal having higher conductance is detected, is indicative that there is protein-DNA interaction.

Step a) of the first method of the invention, comprises putting into contact the protein with a DNA molecule, said DNA molecule comprising a ssDNA region and a dsDNA region, wherein the dsDNA region contains the DNA target sequence, with a planar lipid bilayer containing at least one alpha hemolysin nanopore under conditions allowing the interaction between the protein, the DNA molecule and the planar lipid bilayer.

The DNA molecule comprises a ssDNA region, which is considered as a DNA reporter sequence, and a dsDNA region containing at least one DNA binding sequence of the target protein.

As used herein, the term "molecule" is used for simplification reasons, and refers, depending on the context, to the nucleic acid and/or amino acid sequences disclosed herein. The skilled worker will know from the context what they refer to. The term "DNA molecule" as used herein is understood as meaning portions of the DNA. As used herein the term "ssDNA" means single-stranded DNA; "dsDNA" means a double-stranded DNA.

"dsDNA containing at least one DNA target sequence", as used herein relates to a nucleic acid DNA, in particular dsDNA, having a sequence, the DNA target sequence relate to a DNA binding sequence of the target protein with the ability of binding to a peptide or protein and may involves some sort of molecular complementarity between protein and DNA. DNA recognition by the DNA binding domain can occur at the major or minor groove of DNA, or at the sugar-phosphate DNA backbone

In a more preferred embodiment, the DNA target sequence is dsDNA.

In a preferred embodiment, the dsDNA comprises more than one target sequence. In a more preferred embodiment, the dsDNA comprises at least 1, at least 2, at least 3 or more target sequence.

A protein suitable to be used in the present invention includes any peptides, proteins, e.g., hormones, enzymes, inhibitors, and receptors, antigens, antibodies including antibody fragments, single-chain antibodies and a member of a specific binding pair. Alternatively, the target protein may be a derivative or analog of any such proteins. Specific binding sequences include any sequences having the ability of binding to a specific DNA sequence. The skill person in the art knowns several methods for determining the specific binding DNA sequence for a particular peptide or protein.

In a preferred embodiment, the protein is a transcription factor.

In a preferred embodiment, the dsDNA region comprises a hairpin region.

The term "hairpin DNA" is used to indicate a duplex DNA in a hairpin configuration, being the 5'- and 3'-termini of the sequence complementary and separated by an intervening non-complementary segment. The complementary segments form a double stranded stem and the non-complementary segment form a loop. It includes nucleic acids that also contain moieties other than ribonucleotide moieties, including, but not limited to, modified nucleotides, modified internucleotide linkages, non-nucleotides, deoxynucleotides and analogs of the nucleotides mentioned thereof. "ds hairpin DNA" or ss hairpin DNA mean single-stranded or double stranded hairpin DNA respectively.

In another preferred embodiment, the ssDNA is located at 5'. In another preferred embodiment, the ssDNA is located at 3'. In another preferred embodiment, the DNA molecule comprises a self-complementary 3' region which hybridizes to form the hairpin region and a 5' region comprising the ssDNA. In another preferred embodiment, the DNA molecule comprises a self-complementary 5' region which hybridizes to form the hairpin region and a 3' region comprising the ssDNA.

In another preferred embodiment, the dsDNA contains a hairpin DNA. In another preferred embodiment, the hairpin DNA has a length of at least 10 to 36 pb, preferably 21 bp. In a preferred embodiment, DNA molecule comprises a self-complementary 3' region which hybridizes to form the hairpin region and a 5' region comprising the ssDNA. In a preferred embodiment, DNA molecule comprises a self-complementary 5' region which hybridizes to form the hairpin region and a 3' region comprising the ssDNA.

In another preferred embodiment, the 5'ssDNA region or the 3'ssDNA region has a length of at least 20 to 100 nucleotides, preferably 30 nucleotides.

"Nucleotides" or "Nucleic acids" as used herein mean biopolymers of nucleotides which are linked with one another via phosphodiester bonds (polynucleotides, polynucleic acids). Depending on the type of sugar in the nucleotides (ribose or deoxyribose), one distinguishes the two classes of the ribonucleic acids (RNA) and the deoxyribonucleic acids (DNA). As used herein it relates to any natural or non-natural nucleic acid" and "oligonucleotide" and "polynucleotide" are used interchangeably in the context of the present invention. In the context of the present invention "Natural nucleotides" mean nucleotides that can be purified from natural sources. "Non- natural nucleotides" are defined as those produced using recombinant expression systems and, optionally, purified, chemically synthesized, etc. When appropriate, for example, in the case of chemically synthesized molecules, the nucleic acids can comprise nucleoside analogues such as analogues having chemically modified bases or sugars, modifications of the backbone, etc. A nucleic acid sequence is represented in 5'-3' direction unless indicated otherwise.

In another preferred embodiment, the DNA binding sequence is near the 3'end on the hybridizing dsDNA. In another preference embodiment, the dsDNA sequence has a high melting temperature. Near as used herein refers to from the first base pair to the sixth base pair.

"Melting temperature", as used herein relates to the temperature at which no crystallinity can be detected by wide angle x-ray diffraction. Alternatively, the melting point may be determined via DSC, for example according to the ISO 11357-3:2011 standard. The melting temperature is hereinafter also represented by the symbol "Tm".

"High melting temperature", as used herein refers to a temperature >20 °C over working temperature (temperature where experiments are performed).

In another preferred embodiment, the alpha hemolysin pore within the lipid bilayer has an average internal diameter of the pore of about 1.3 nm.

The term "planar lipid bilayer" is used herein to refer to a thin polar planar membrane made of two layers of lipid molecules, arranged so that the hydrophilic phosphate heads point "out" to the water on either side of the bilayer and the hydrophobic tails point "in" the core of the bilayer. The lipid bilayers are usually a few nanometers in width, and they are impermeable to most charged water-soluble molecules. Lipid bilayers are large enough structures to have some of the mechanical properties of liquids or solids. The area compression modulus Ka, bending modulus Kb, and edge energy, can be used to describe them. Solid lipid bilayers also have a shear modulus, but like any liquid, the shear modulus is zero for fluid bilayers. Lipid bilayers can also be supported by solid substrates having apertures, such as heat shrink tubing, fused silica, borosilicate glass, mica, and oxidized silicon. Lipids may be applied, e.g., through Langmuir-Blodgett technique, vesicle fusion processes or the combination of the two. In a preferred embodiment, the lipid bilayer is formed by diphytanoyl-sn-glycero-3-phosphocholine.

"Hemolysin nanopore", as used herein is used to refer to any small hole or channel of the order of 0.5 to 10 nanometers in internal diameter. The term "nanopore" includes both biological (e.g. α-hemolysin) or artificial nanopores. The present nanopores can vary in dimensions, for example it can have a diameter of between about 0.5 nm and 10 nm in size. For example, the diameter can be about 0.5 nm, 1 nm, 1.25 nm, 1.5 nm, 1.75 nm, 2 nm, 2.25 nm, 2.5 nm, 2.75 nm, 3 nm, 3.5 nm, 4 nm, 4.5 nm, 5 nm, 6 nm, 7 nm, 8 nm, 9 nm, 10 nm, or any dimension there between. Biological nanopores can be created by pore proteins. Artificial nanopores can be made by micromolding or drilling. They also can be made by etching a somewhat larger hole (several tens of nanometers) in a piece of silicon, and then gradually filling it in using ion-beam sculpting methods which results in a much smaller diameter hole.

The term "α-hemolysin" is used herein to refer to a pore-forming toxin from the bacterium, *Staphylococcus aureus.* α-hemolysin consists mostly of beta-sheets (68%) with only about 10% alpha-helices. The hla gene on the *S. aureus* chromosome encodes the 293 residue protein monomer, which forms heptameric units on the cellular membrane to form a complete beta-barrel pore. This structure allows the toxin to perform its major function, development of pores in the cellular membrane.

In another preferred embodiment, the alpha hemolysin pore within the lipid bilayer has an average internal diameter of the pore of about 1.3 nm.

A skilled person in the art knows several methods for isolating hemolysin nanopores. Either using the protein from natural sources or by recombinant expression, which can be either with affinity tags or without them. As a way of illustrative non limitative examples, the method described in the experimental part of the description may be used.

Step b) of the first method of the invention comprises applying a positive electric potential across the lipid bilayer which is capable of electrophoretically driving the ssDNA through the alpha-hemolysin nanopore within the lipid bilayer.

"Positive electric potential" as used herein, relates to the trans-membrane potential defined as the electric potential difference between the bulk aqueous phases at the two sides of the membrane and results from the selective transport of charged molecules across the membrane. As a rule, the potential at the cytoplasm side of cell membranes is negative relative to the extracellular physiological solution.

A skilled person in the art knows several method for applying a positive electric potential, such as a way of illustrative non limitative example, either from electrodes connected to an amplifier or by generating a electrochemical gradient across the membrane. In a particular aspect, the positive electric potential is applied as described in the experimental part of the application.

Step c) of the first method comprises measuring the ionic current through the alpha-hemolysin nanopore. Step c) of the first method of the invention, allows detecting if the ssDNA reporter sequence passes through the alfa hemolysin nanopore and the dsDNA is outside the pore.

Alternatively, an initial level in the ionic current signal having higher conductance is detected, is indicative that there is protein-DNA interaction. Said higher conductance is considered in comparison with the ionic current signal obtained in the absence of the protein. Said level that can be modulated by changing the sequence of the ssDNA - reporter.

According to the first method of the invention, an interaction between the protein and the DNA target sequence is detected if the decrease in conductance after the application of the electric potential is reduced at least transiently when compared with the decrease in conductance in the absence of the protein

Method for identifying a compound capable of modulating protein-DNA interaction

In a second aspect, the invention relates to a method for identifying a compound capable of modulating the interaction between a protein and a DNA target sequence, second method of the invention, comprising:

a) putting into contact the compound with the protein, a DNA molecule, said DNA molecule comprising a ssDNA region and a dsDNA region, wherein the dsDNA region contains the DNA target sequence and with a planar lipid bilayer containing at least one alpha hemolysin nanopore, under conditions allowing the interaction between the compound, the protein, the DNA molecule and the planar lipid bilayer,

b) applying a positive electric potential across the lipid bilayer which is capable of electrophoretically driving the ssDNA through the alfa hemolysin nanopore within the lipid bilayer, and

c) measuring the ionic current through the alpha-hemolysin nanopores,

wherein if an initial level in the ionic current signal having higher conductance is detected in comparison with the signal in the absence of said compound, is indicative the compound is an enhancer of the protein-DNA interaction, and wherein if an initial level in the ionic current signal having higher conductance is detected not in comparison with the signal in the absence of said compound, is indicative the compound is an inhibitor of the protein-DNA interaction.

In a preferred embodiment, the dsDNA comprises more than one target sequence. In a more preferred embodiment, the dsDNA comprises at least 1, at least 2, at least 3 or more target sequence.

In a preferred embodiment, the protein is a transcription factor.

In a preferred embodiment, the dsDNA comprises a hairpin DNA. In a preferred embodiment, the DNA molecule comprises a self-complementary 3' region which hybridizes to form the hairpin region and a 5' region comprising the ssDNA. In a preferred embodiment, the DNA molecule comprises a self-complementary 5' region which hybridizes to form the hairpin region and a 3' region comprising the ssDNA.

In a preferred embodiment, the alpha hemolysin pores within the lipid bilayer has an average internal diameter of the pore of about 1.3 nm. In another preferred embodiment, the hairpin DNA has a length of at least 10 to 36 bp, preferably 21 bp. In another preferred embodiment, the 5'ssDNA region has a length of at least 20 to 100 nucleotides, preferably 30 nucleotides. In another preferref embodiment, the e binding sequence of the target protein is near the 3'end on the hybridizing dsDNA.

In a preferred embodiment, "near" the 3'end on the hybridizing dsDNA means from the first base pair to the sixth.

In another preference embodiment, the the dsDNA sequence has a high melting temperature.

In a preferred embodiment, the lipid bilayer is formed by diphytanoyl-sn-glycero-3-phosphocholine.

In a preferred embodiment, higher melting temperature means at least 20 degrees above the working temperature.

"Method for identifying a compound capable of modulating protein-DNA interaction" as used herein, relates to a method for determining whether a compound is capable of inhibiting the protein-DNA interaction or is capable of enhancing said interaction. Inhibiting the interaction, as used herein relates to a reduction in the activity by at least 5 percent, 10 percent, 15 percent, 20 percent, 25 percent, 30 percent, 35 percent, 40 percent, 45 percent, 50 percent, 60 percent, 70 percent, 80 percent, 85 percent, 90 percent, or at least 95 percent compared to the activity in the absence of the compound to be tested, or compared to a reference value. Alternatively, enhancement of the activity, as used herein relates to an increase in the activity by at least 5 percent, 10 percent, 15 percent, 20 percent, 25 percent, 30 percent, 35 percent, 40 percent, 45 percent, 50 percent, 60 percent, 70 percent, 80 percent, 85 percent, 90 percent, or at least 95 percent compared to the interaction in the absence of the compound to be tested or compared to a reference value.

"Reference value", as used herein relates to a laboratory value used as a reference for the values/data obtained from samples. The reference value (or reference level) can be an absolute value, a relative value, a value which has an upper and/or lower limit, a series of values, an average value, a median, a mean value, or a value expressed by reference to a control or reference value. A reference value can be based on the value obtained from an individual sample, such as, for example, a value obtained from a sample of study but obtained at a previous point in time. The reference value can be based on a high number of samples, such as the values obtained in a population of samples or based on a pool of samples including or excluding the sample to be tested. In a particular embodiment, the reference value is the ionic current signal obtained in the absence of protein, which means the ionic current signal analyzing the driving of the ssDNA of the DNA molecule through the alpha-hemolysin nanopore within the lipid bilayer.

"Compound" to be tested according to the present invention may be a chemical compound or a biological product obtained by chemical synthesis and/or isolated from a live or dead organism, and therefore includes native compounds or synthetic compounds, and derivatives thereof. Examples of candidate compounds include drugs, molecules with affinity and selectivity for the previously described components involved in the mitochondrial oxidative phosphorylation process or any of the proteins that modulate its function, including antisense antibodies or fragments thereof.

The term includes, for example, polynucleotides, DNA, RNA, polypeptides, proteins, and combinations thereof. The compound may be naturally occurring or may be engineered or synthetic.

In one process for molecular analysis, it has been shown that molecules such as nucleic acids and proteins can be transported through a natural or synthetic nano-scale pore, or "nanopore", and that characteristics of the molecule, including its identification, its state of hybridization, its interaction with other molecules, its sequence, i.e., the linear order of the monomers of which a polymer is composed, can be discerned by and during transport through the nanopore. Transport of a molecule through a nanopore can be accomplished by, e.g., electrophoresis, or other translocation mechanism. If the dimensions of the nanopore are such that an extended nucleic acid molecule occupies a substantial fraction of the nanopore's cross-sectional area during translocation, the polymer molecule can be characterized by and during transport through the nanopore by at least two mechanisms. In a first of these, the translocating molecule transiently reduces or blocks the ionic current produced by application of a voltage between the two compartmentalized liquid ion-containing solutions in contact with each end of nanopore. In a second of these, the translocating molecule transiently alters the electron current, including the tunneling electron current, produced by applying a bias between two closely spaced local probes that are located to produce a nanoscale gap, either on apposed points on the perimeter of the nanopore or at opposite ends of a very short nanopore. Given that during its passage through the nanopore each nucleotide in the polymer produces a characteristically distinct modulation of the ionic current or the electron current, the resulting sequence of either the ionic or the electron current modulations can reflect the characteristics of the translocating polymer molecule.

According to the second method of the invention, if an initial level in the ionic current signal having higher conductance is detected in comparison with the absence in said compound, is indicative the compound is an enhancer of the protein-DNA interaction, and wherein if an initial level in the ionic current signal having higher conductance is not detected in comparison with the absence in said compound, is indicative the compound is an inhibitor of the protein-DNA interaction.

Alternatively, if the compound is an enhancer of the interaction between a protein and a DNA target sequence there is a decrease in conductance after the application of the electric potential is reduced at least transiently when compared with the decrease in conductance in the absence of the protein, and the compound is an inhibitor of the interaction between a protein and a DNA target sequence if a decrease in conductance after the application of the electric potential is not reduced at least transiently when compared with the decrease in conductance in the absence of the protein

All the terms and embodiments previously described are equally applicable to this aspect of the invention.

Kit of the invention and uses

In another aspect, the invention relates to a kit comprising at least one protein, at least one DNA molecule, said DNA molecule comprising a ssDNA region and a dsDNA region, wherein the dsDNA region contains the DNA target sequence, and a planar lipid bilayer containing at least one alpha hemolysin nanopore.

In a preferred embodiment, the dsDNA region comprises a hairpin DNA. In another preferred embodiment, the dsDNA region comprises one or more DNA target sequence.

In a preferred embodiment, the DNA sequence comprises a self-complementary 3'region that hybridizes into a hairpin dsDNA followed by a 5'ss DNA overhang. In a preferred embodiment, the DNA sequence comprises a self-complementary 5'region that hybridizes into a hairpin dsDNA followed by a 3'ss DNA overhang. In another preferred embodiment, the hairpin DNA has a length of at least 10 to 36 bp, preferably 21 bp. In another preferred embodiment, the 5'ssDNA region has a length of at least 20 to 100 nucleotides, preferably 30 nucleotides. In another preferred embodment, the alpha hemolysin pores within the lipid bilayer has an average internal diameter of the pore of about 1.3 nm

In the context of the present invention, "kit" is understood as a product containing the different reagents necessary for carrying out the methods of the invention packed so as to allow their transport and storage. Additionally, the kits of the invention can contain instructions for the simultaneous, sequential or separate use of the different components which are in the kit. Said instructions can be in the form of printed material or in the form of an electronic support capable of storing instructions susceptible of being read or understood, such as, for example, electronic storage media (e.g. magnetic disks, tapes), or optical media (e.g. CD-ROM, DVD), or audio materials. Additionally or alternatively, the media can contain internet addresses that provide said instruction.

The kit of the invention may further comprise an assay buffer.

In a preferred embodiment, the target protein, the DNA sequence an alfa hemolysin nanopore comprise at least 10%, at least 20%, at least 30%, at least 40%, at least 50%, at least 60%, at least 70%, at least 80%, at least 90% or at least 100% of the total amount of reagents forming the kit.

In a preferred embodiment, the DNA molecule comprises a self-complementary 3' region which hybridizes to form the hairpin region and a 5' region comprising the ssDNA. In another preferred embodiment, the DNA molecule comprises a self-complementary 5' region which hybridizes to form the hairpin region and a 3' region comprising the ssDNA.

In a preferred embodiment, the dsDNA sequence has a high melting temperature. In a preferred embodiment, the dsDNA comprises more than one target sequence. In a more preferred embodiment, the dsDNA comprises at least 1, at least 2, at least 3 or more target sequence.

In a preferred embodiment, the protein is a transcription factor.

In another preferred embodiment, the lipid bilayer is formed by diphytanoyl-sn-glycero-3 -pho sphocho line

In another aspect, the invention relates to the user of a kit of the invention for detecting protein-DNA interaction and/or for identifying a compound capable of modulating protein-DNA interaction.

All the terms and embodiments previously described are equally applicable to this aspect of the invention.

The invention will be described by way of the following examples which are to be considered as merely illustrative and not limitative of the scope of the invention.

## Examples

## Materials and methods

## Preparation of hemolysin pores

BL21 (DE3) cells (Novagen) were transformed with a pT7 vector containing the WT $\alpha$-HL gene.[46] Single colonies were grown 16 h at 25 °C in 50 mL of LB medium supplemented with 0.1 mg/mL ampicillin under shaking. The grown cultures were centrifuged and the cell pellet was suspended in 0.5 L of fresh LB medium supplemented with 0.1 mg/mL ampicillin and shaken at 25 °C for 2 h. This culture was induced with 1 mM IPTG final concentration and shaken at 25 °C for 4 h. After this, the cells were harvested by centrifugation and suspended in 30 mL of 30 mM Tris·HCl, 50 mM EDTA pH 8.0. Cells were lysed by sonication and the lysate centrifuged at 100,000 x g for 45 min. Ammonium sulphate was added to the supernatant to 75 % saturation and stirred for 1 h at 4 °C. The solution was centrifuged 50 min at 40,000 x g and the pellet suspended in 5 mL of 20 mM NaCl, 10 mM sodium acetate pH 5. After overnight dialysis against the same buffer to remove the ammonium sulphate (4 °C) the suspension was centrifuged 40,000 x g for 50 min. The supernatant was loaded in a Superdex 200 16/60 (Pharmacia) equilibrated in the same buffer. The peak corresponding to the monomeric $\alpha$-HL protein was identified by SDS-PAGE.

Rabbit erythrocyte membranes were prepared from fresh blood. Approximately 100 mL of blood was mixed with 50 mL of citrate anticoagulant (80 mM citrate, 140 mM dextrose, pH 7.4). The red blood cell fraction was purified by centrifugation (500 x g 10 min) and washed with PBS (150 mM sodium chloride, 10 mM sodium phosphate, pH 7.4) three times. Erythrocytes were lysed by 1:1000 dilution in hypotonic solution (5 mM HEPES pH 7.4), drop by drop, under stirring and at 4 °C. Keeping the resulting solution at 4 °C, cell membranes were precipitated from the cell content by centrifugation at 20,000 x g and washed against the same buffer three further times. A large fraction of the cytoskeleton remains bound to the membrane, it was removed by diluting the pellet of membranes in 2 M NaCl, 10 mM HEPES pH 7.4 and centrifuged (three times, 20,000 x g, 4 °C). The final pellet was suspended and stored at -20 °C.

$\alpha$-HL heptamers were obtained incubating 50 $\mu$L of the previously purified rabbit erythrocyte membranes with 250 $\mu$L of monomers (0.3 mg/mL) for 3 min at room temperature. Membranes containing the heptamers were purified by centrifugation (20,000 x g 5 min) and washed 3 times with 2 M NaCl, 10 mM HEPES pH 7.4. The final pellet was suspended with 5 $\mu$L 10% SDS (w/v), 45 $\mu$L 2 M NaCl 10 mM HEPES, pH 7.4 and incubated 20 min at room temperature to extract the pores from the membrane. The solution was centrifuged once more 20,000 x g and the supernatant containing the heptamers were stored at -80 °C.

## Purification of DNA-binding proteins

## Replication factor E

RepE and RepE54 proteins were purified from an intein vector pTXB1 containing the ORF fused with intein at the C terminus, followed by the chitin binding domain. BL21 (DE3) (Novagen) cells were transformed with the vector, grown in LB medium supplemented with 0.1 mg/mL ampicillin, and when the O.D. reached 0.6 the cells were induced with 0.4 mM IPTG. The fusion protein was overexpressed for 3 h at 30 °C after induction. After lysis and centrifugation the supernatant was incubated with chitin-beads (New England Biolabs) 12 h at 4 °C in buffer A: 20 mM Tris·HCl pH 7.5, 400 mM KCl, 10% (v:v) Glycerol, 1 mM EDTA. To elute the protein, the resin was incubated overnight at 4 °C in the same buffer supplemented with 50 mM DTT to induce the cleavage of the intein fused protein. The supernatant was injected in a HiTrap SP column (GE Healthcare), eluted with a KCl gradient (from 0.4 to 1 M in buffer A), followed by gel filtration in a Superdex 75 (GE Healthcare), equilibrated in buffer A. The fractions containing the protein were verified by SDS-PAGE and stored at -20 °C.

**Forkhead box protein M1**

Forkhead box protein M1C was purified as published.[38] Briefly, the FoxMIC gene (residues 222-360) was cloned in a pET-28 vector fused to the N-terminal tag MAHHHHHHSAALEVLFQGPG. (SEQ ID NO: 1) BL21 (DE3) (Novagen) cells were transformed and grown in LB medium supplemented with 0.1 mg/mL ampicillin at 37 °C until reaching an OD ~ 0.8-1.0. Protein expression was induced for 16 h at 15 °C by adding 0.5 mM IPTG. The medium was centrifuged and cells were resuspended in buffer A (20 mM Tris·HCl pH 8.0, 500 mM NaCl, 20 mM imidazole, 0.5 mM TCEP). Following sonication and centrifugation, the supernatant was loaded on a Ni-NTA metal affinity column (GE Healthcare) and the protein was eluted with a 20 to 400 mM imidazole gradient. The N-terminal tag was cleaved incubating the protein overnight with human rhinovirus 3C protease (Sigma-Aldrich). The product was loaded on a Superdex G75 16/60 HiLoad size exclusion column (GE Healcare) equilibrated in buffer A. The fractions containing the protein were verified by SDS-PAGE, concentrated and stored at -20 °C.

**Primer design**

DNA oligonucleotides were purchased from Biomers (Ulm/Donau, Germany). The followed design was the same for all the DNAs. Oligonucleotides were composed of a 30 nt-long 5'single-stranded overhang, formed by two different segments that act as reporters: poly(dA)/poly(dC) or poly(dC)/poly(dA). In 3' region the sequence self-hybridized forming a hairpin-like dsDNA. This dsDNA region contained the binding sequence for RepE or for FOXM1C[38]. Sequences employed in this work were

RepE (DIRECT) SEQ ID NO: 2

5' AAA AAA AAA AAA AAA AAA AAC CCC CCC CCC CCT GTG ACA AAT TGC CCT CAG CAC ACA CTG AGG GCA ATT TGT CAC AGG 3'

RepE (INVERSE) SEQ ID NO: 3

5' CCC CCC CCC CCC CCC CCC CCA AAA AAA AAA ACT GTG ACA AAT TGC CCT CAG CAC ACA CTG AGG GCA ATT TGT CAC AGG 3'

FOXM1 SEQ ID NO: 4

5' AAA AAA AAA AAA AAA AAA AAC CCC CCC CCC AAA TTG TTT ATA AAC AGC CCG CAC ACA CGG GCT GTT TAT AAA CAA TTT 3'

RepE (LevelP) SEQ ID NO: 5

5'AAA AAA AAA AAA AAA AAA AA -Spacer*- CCC CCC CCC TGT GAC AAA TTG CCC TCA GCA CAC ACT GAG GGC AAT TTG TCA CAG 3'

Mismatched SEQ ID NO: 6

5' AAA AAA AAA AAA AAA AAA AAC CCC CCC CCC CCT CTG ACA AAT TGC CCT CAG CAC ACA CTG AGG GCA ATT TGT CAC AGG 3'

Disrupted RepE binding site SEQ ID NO: 7

5' AAA AAA AAA AAA AAA AAA AAC CCC CCC CCC CCT CTG ACA AAT TGC CCT CAG CAC ACA CTG AGG GCA ATT TGT CAG AGG 3'

\* Spacer = hexaethylene glycol

Note that in the RepE (INVERSE) the first G at the 3' end does not hybridize. We found that if this base-pair is formed the unzipping times are too long, compromising the collection of thousands of events in the multiplexed experiments (see Figure 15).

**Single-molecule measurements and data analysis**

Single-molecule measurements were performed in planar lipid bilayers made of 1,2-diphytanoyl-*sn*-glycero-3-phosphocholine (Avanti Polar Lipids) constructed with the Montal and Mueller method across an aperture in a Teflon film (0.025 mm thick, Goodfellow) of 50-100 $\mu$m in diameter that separated the two compartments of a chamber (*cis* and *trans*).[11] The measurements were recorded at 22 $\pm$ 1.5 °C in asymmetric conditions. The *cis* compartment (connected to the ground electrode and where samples were added) was filled with 5 mM MgCl$_2$, 150 mM KCl, 10 mM HEPES pH 7.2 (to mimic physiological conditions) and the *trans* compartment with 5 mM MgCl$_2$, 2 M KCl, 10 mM HEPES pH 7.2 (to increase the ionic current signal). 0.5 $\mu$L of previously heptamerized $\alpha$-HL was added to the *cis* compartment and following the first insertion the chamber was manually perfused to remove excess of $\alpha$-HL. Ionic currents through the $\alpha$HL pore were measured using Ag/AgCl electrodes with agar bridges in 3 M KCl, and balanced before measurements. The electrodes were connected to an amplifier (Axopatch 200B, Molecular Devices), the signal was filtered at 5 kHz (lowpass Bessel filter) and acquired at 20 kHz with a digitizer (Digidata 1440A, Molecular Devices). All experiments were repeated in at least 3 different pores. Raw data was first analyzed with pClamp software (Molecular Devices) to obtain the current and dwell time of the ionic current levels, >90% of the signals longer than 10 ms were the characteristic signals described in the main text. The residual current of a level is defined as the ionic current of the level divided by the ionic current of the open pore. Data analysis was carried out with Igor Pro (Wavemetrics) and molecular representations were performed using Chimera (UCSF).[47] The confidence interval of the kinetic rates were estimated using:

$$k_{upp} = k \cdot (1 + z/\sqrt{n})$$

$$k_{low} = k \cdot (1 - z/\sqrt{n})$$

With k = kinetic constant; z = significance level and n = number of data. The histograms of dwell times were made in Igor Pro with bin width 1.1 (note that natural logarithm scale was used in the time axis). The histograms representing the probability density function (pdf) were fitted to a mono -or double exponential-:

$$\text{mono exponential pdf}(x) = A \cdot k \cdot \exp(x - k \cdot \exp(x))$$

with A being the amplitude, k the rate and x the natural logarithm of the dwell time.

**Data analysis of multiplexed experiments**

The multiplexed experiments were performed and analysed as follows: First the oligonucleotide containing the FOXM1 binding site (oligo-FOX) and the protein FOXM1 were analysed alone to define the residual current at levels P and D and the dwell time distribution at level D. Next, the oligonucleotide containing the RepE binding site (oligo-RepE) and the protein RepE were added at half the concentration of FOXM1 and its oligonucleotide. Then the sample was subjected to nanopore analysis in the absence and at increasing concentrations of the inhibitor FDI-6. Data from all conditions was analysed together to obtain the distribution of residual currents at level D for oligo-FOX and oligo-RepE, the distribution of residual currents at level P for oligo-FOX bound to protein and oligo-RepE bound to protein and the distribution of dwell times at level D for oligo-FOX and oligo-RepE. Data were binned and normalized by the total number of events to obtain the frequency (with equal number of bins for each data set). Next, residual current histograms were fitted to normal distributions to obtain the means and standard deviations ($I_{D,RepE}$, $S_{D,RepE}$; $I_{D,FOX}$, $S_{D,FOX}$; $I_{P,RepE}$, $S_{P,RepE}$; $I_{P,FOX}$, $S_{P,FOX}$), and the dwell time histogram to two exponential distributions to obtain the inverse of the characteristic times ($t_{D,RepE}$ and $T_{D,FOX}$; Figure 11). The protein bound species were assigned to RepE-bound events or FOXM1-bound events based on their particular values of $I_P$, $I_D$ and the inverse of the dwell time at level D ($t_D$) following the rule:

$$\text{RepE if } P_T > 0$$

$$\text{FOXM1 if } P_T < 0$$

where $P_T$ is defined as:

$$P_T = (w_P \cdot (P_{IP,RepE} - P_{IP,FOXM1}) / (P_{IP,RepE} + P_{IP,FOXM1}) + w_D \cdot (P_{ID,RepE} - P_{ID,FOXM1}) /$$

$$(P_{ID,RepE} + P_{ID,FOXM1}) + w_{tD} \cdot (P_{tD,RepE} - P_{tD,FOXM1}) / (P_{tD,RepE} + P_{tD,FOXM1})) / (w_P + w_D + w_{tD})$$

and

$$P_{IP,RepE} = (1/\sqrt{(2 * \pi * S_{P,RepE}\hat{}2)}) * \exp(-(I_P - I_{P,RepE})\hat{}2/(2 * S_{P,RepE}\hat{}2))$$

$$P_{IP,FOXM1} = (1/\sqrt{(2 * \pi * S_{P,FOXM1}\hat{}2)}) * \exp(-(I_P - I_{P,FOXM1})\hat{}2/(2 * S_{P,FOXM1}\hat{}2))$$

$$P_{ID,RepE} = (1/\sqrt{(2 * \pi * S_{D,RepE}\hat{}2)}) * \exp(-(I_D - I_{D,RepE})\hat{}2/(2 * S_{D,RepE}\hat{}2))$$

$$P_{ID,FOXM1} = (1/\sqrt{(2 * \pi * S_{D,FOXM1}\hat{}2)}) * \exp(-(I_D - I_{D,FOXM1})\hat{}2/(2 * S_{D,FOXM1}\hat{}2))$$

$$P_{tD,RepE} = t_{D,RepE} * \exp(\ln(t_D) - t_{D,RepE} * \exp(\ln(t_D)))$$

$$P_{tD,FOXM1} = t_{D,FOXM1} * \exp(\ln(t_D) - t_{D,FOXM1} * \exp(\ln(t_D)))$$

with

$$w_P = \log_{10} \chi^2 \text{ (chi-square of the fit in residual current distribution of level P)}$$

$$w_D = \log_{10} \chi^2 \text{ (chi-square of the fit in residual current distribution of level D)}$$

$$w_{tD} = \log_{10} \chi^2 \text{ (chi-square of the fit in dwell time distribution at level D)}$$

the non-bound events were assigned to oligo-FOX or to oligo-RepE following the rule:

$$\text{oligo-RepE if } P_{TI} > 0$$

$$\text{oligo-FOX if } P_{TI} < 0$$

where $P_{TI}$ is defined as:

$$P_{TI} = (w_D \cdot (P_{ID,RepE}-P_{ID,FOXM1})/ \quad (P_{ID,RepE}+P_{ID,FOXM1})+w_{tD} \cdot (P_{tD,RepE}-P_{tD,FOXM1})/$$

$$(P_{tD,RepE}+P_{tD,FOXM1}))/( w_D + w_{tD})$$

and

$$P_{ID,RepE} = (1/\sqrt{(2*\pi* S_{D,RepE}^2)})*exp(-(I_D- I_{D,RepE})^2/(2* S_{D,RepE}^2))$$

$$P_{ID,FOXM1}= (1/\sqrt{(2*\pi* S_{D,FOXM1}^2)})*exp(-(I_D- I_{D,FOXM1})^2/(2* S_{D,FOXM1}^2))$$

$$P_{tD,RepE} = t_{D,RepE}*exp(ln(t_D)- t_{D,RepE}*exp(ln(t_D)))$$

$$P_{tD,FOXM1}= t_{D,FOXM1}*exp(ln(t_D)- t_{D,FOXM1}*exp(ln(t_D)))$$

with

$$w_D = log_{10} \chi^2 \text{ (chi-square of the fit in residual current distribution of level D)}$$

$$w_{tD} = log_{10} \chi^2 \text{ (chi-square of the fit in dwell time distribution at level D)}$$

**Anisotropy Fluorescence**

20 nM of a dsDNA fragment tagged with FITC at the 5' end of one of the strands and containing the binding site of RepE was incubated with increasing concentrations of RepE wild type (0.001-20 $\mu$M) 12 h at 4 °C in 20 mM HEPES pH 7.4, 200 mM KCl, 5 mM MgCl$_2$. Fluorescence anisotropy intensity measurements were performed in a Fluorolog spectrofluorimeter (Jobin Ibon) with excitation and emission wavelengths at 494 nm and 516 nm, respectively using 5 nm slits widths.

EXAMPLES

The following examples are provided to illustrate further the various applications and are not intended to limit the invention beyond the limitations set forth in the appended claims.

**Example 1- Nanopore characterization of specific protein-DNA complexes.**

To detect sequence-specific protein interactions we base our approach on an oligonucleotide design with a self-complementary 3' region that hybridises into a hairpin dsDNA of 21 bp followed by a 5' ssDNA overhang of 30 nt (Figure 1a, Figure 12). This construct is electrophoretically driven through a single $\alpha$-HL pore inserted in a planar lipid bilayer when a positive electric potential is applied across the membrane. We carry out our experiments in asymmetric conditions with the sample container (cis side) filled with physiological buffer (150 mM KCl, 5 mM MgCl$_2$, 10 mM HEPES, pH 7.2) and a small amount of the DNA construct (0.04-0.2 $\mu$M), and the recipient container (trans side) filled with a high salt buffer to increase the ionic current signal (2 M KCl, 5 mM MgCl$_2$, 10 mM HEPES, pH 7.2). As the construct threads through the pore we obtain a characteristic ionic current signal (Figure 1a). In agreement with previous findings,[12] this signal represents the threading of the ssDNA (level D) with the dsDNA in the vestibule of the pore. Because the internal diameter of the pore stem channel is only 1.3 nm, the double stranded portion of the DNA cannot translocate through this stem until de dsDNA unzips. The dwell time at level D is the time it takes to unzip the DNA under the constant electrophoretic force acting on the ssDNA in the pore stem.[12] We observe that after unzipping the previously base-paired double stranded portion of the DNA completes translocation through the pore (level T, Figure 1a), this level is only observed when asymmetric conditions are used. More important and as previously reported,[12,29] the unzipping times depend exponentially on the applied voltage (from $k_{unz,120mV}$ = 1.8 [3 - 0.6] s$^{-1}$ to $k_{unz,100mV}$ = 0.12 [0.2 - 0.04] s$^{-1}$; 95% Confidence Interval -CI- in brackets; n = 7; Figure 5a,b) and if a single mismatch is introduced at the fourth base pair in the dsDNA the unzipping time is reduced ($k_{unz,120mV,mis}$ = 96 [162 - 30] s$^{-1}$; n = 3; Figure 12 and Figure 5c).

The dsDNA is designed to contain the binding sequence of the Replication Factor E (RepE) near the 3' end on the hybridising dsDNA (Figure 12), which regulates the replication of the bacterial miniF plasmid (Figure 1b). When the DNA is analysed in the presence of RepE proteins, we detect some events that show an initial new level, P, in the ionic current signal (Figure 1b). The number of such events depends on the amount of protein that is added: in a titration of 0.041 μM DNA with 0.05, 0.12, 0.36, 0.72 and 1.2 μM RepE 7%, 46%, 54%, 66% and 80% of the DNA molecules translocating the pore show the P level (this initial observation was further confirmed 7 times, n = 8). We therefore deduce that the P level represents a state in which the protein is bound to its target sequence atop the α-HL pore, because the dsDNA-protein complex is too big to enter the vestibule through the 2.3-nm-wide entrance. Once the protein is detached the dsDNA enters the vestibule and the ionic current signal proceeds through levels D and T (Figure 1b, Figure 12, Figure 6). During level P, the pore is filled with ssDNA and the dsDNA is outside the pore, instead during level D the dsDNA enters the vestibule and is in contact with the pore constriction. This two different configurations cause the two different ionic current levels P and D, where P shows higher conductance (see Figure 12 for details on the proposed model). The time in level D is the time it takes to unzip the dsDNA and it proceeds with a rate $k_{unz,120mV}$ = 2.6 [4.4 - 0.8] s$^{-1}$, similar to the unzipping in the absence of protein (95% C.I.; n = 7; Figure 5d). In addition, a similar ionic current signature was found by Benner *et al.* upon DNA polymerase detachment from a dsDNA-polymerase complex formed atop the α-HL nanopore.[18] To test whether the RepE-dsDNA interaction was sequence specific we change a C-G base pair to G-C in a position involved in RepE binding. In this case, we have to use higher protein concentrations (2.1 μM) to observe protein-bound DNA molecules (5.6% of the molecules translocating the pore, experiment repeated twice, n = 2), probing the expected lower affinity for the mutated binding sequence.

In a typical experiment, both free DNA molecules and DNA-protein complexes are detected (Figure 2a). This allows estimates of the fractions of bound and free DNA molecules, from which the $K_d$ is calculated (0.34 ± 0.08 μM, mean ± S.D., n = 8). We used an independent method, fluorescence anisotropy, to estimate the $K_d$, and obtained a similar value of 0.23 ± 0.03 μM (repeated twice, 1σ error in the parameter estimation of the fit, Figure 2b-e). We performed our measurements over a range of voltages (from +90 to +120 mV) and protein:DNA ratios and we observed that the $K_d$ value did not depend on them (Figure 2e). Rather, the relevant factors to obtain a good $K_d$ estimate were the number of events measured (n) and the fraction of bound (p) and unbound (q) DNA (Figure 2d,e). The values of p and q can be monitored in real time, which allows to estimate the $K_d$ in a single experiment (*i.e.* no need to obtain a binding curve) as the experiment is running with confidence intervals calculated with the Wilson Score method (note that this error does not include experimental variability). Importantly, we observed that the frequency of events decreased at higher protein concentrations. Nonetheless, we found that it had no effect on the $K_d$ estimation suggesting that both bound and free oligonucleotides are captured with the same efficiency (Figure 7).

We next examined the life-time of the protein-bound state. Protein detachment ($k_{off}$) showed a significant voltage dependence (Figure 8), which suggests that the applied force contributes to the dissociation rate (note that the oligonucleotide threaded in the nanopore is subjected to an electrophoretic force). According to our model, the protein would be "peeled off" as the dsDNA is forced to translocate through the entrance atop the pore. An experimental determination of the forces exerted in nanopore experiments has recently suggested that +160 mV would exert a force of 10 pN on the DNA-protein complex,[30] in the range of those used by several molecular machines.[31] We observed that the dissociation time distribution data was better fitted to two populations with $k_{off1,+120mV}$ = 256 [423 - 88.7] s$^{-1}$ and $k_{off2,+120mV}$ = 17 [28 - 6] s$^{-1}$, which suggests two different protein-bound states (95% CI, Figure 2f and Figure 8). Indeed, it has been reported that RepE binds DNA both as monomer and as a dimer.[3] Each state has a different function: monomeric RepE promotes plasmid replication whereas dimeric RepE inhibits it. The two conformations are not in equilibrium but their ratio is controlled by the DnaK chaperone system and in its absence most of the protein is found as dimer.[3] Both the structure of the dimer and of the monomer bound to the target sequence have been solved.[2,32] Upon dimerization, the protein rearranges one of its DNA-binding domains, and the resulting dimer has fewer contacts with DNA. To assign each population in the dwell time distribution we used the naturally occurring mutant RepE54 because it activates plasmid replication and only exists as a monomer.[3] The release times of this mutant showed a single population with a $k_{off}$ similar to that of the lowest populated state of wild type RepE bound to DNA ($k_{off,+120mV}$ = 8 [14 - 2] s$^{-1}$; 95 % CI; n = 3; Figure 2f). We therefore suggest that the population dissociating more slowly through the "peeling" mechanism corresponds to the monomeric form, and that the slower dissociation is likely caused by a higher number of protein-DNA contacts. While it is possible to extrapolate the detachment rate at cero voltage to obtain information on how it proceeds in solution, the results should be taken cautiously as they represent at best a lower bound (*i.e.* it may proceed faster but is unlikely to proceed slower, Figure 8). Nonetheless, the mechanism inducing protein detachment in our approach may have implications *in vivo.* For example, optical tweezers have been successfully used to induce protein detachment by unzipping DNA as may occur in the RecBCD system during DNA unzipping.[33,34] Instead, the "peeling" mechanism used in our approach resembles the mechanism used by some helicases and translocases to produce protein detachment from DNA.[35,36]

**Example 2-Evaluating inhibitors of Transcription Factor binding.**

Transcription factors are frequently considered as ideal targets for cancer therapy, yet finding molecules capable of specifically inhibiting their interactions with DNA is challenging. Motivated by the high sensitivity of our measurements, we aimed to explore the capabilities of the nanopore to detect small molecule activity. We choose the DNA binding domain of Forkhead box protein M1 (FOXM1), which is overexpressed in many cancers and for which a molecule that inhibits binding to its target sequence has been reported, FDI-6[37] (Figure 3). When a DNA hairpin containing the binding sequence[38] and a 5' overhang is subjected to nanopore analysis we observe the characteristic ionic current signal reflecting the threading, unzipping and translocation of the DNA (Figure 3a). While the dsDNA region of this oligonucleotide contains the same number of base pairs as the RepE oligonucleotide design, its unzipping is faster ($k_{unz,FOXM1,100mV}$ = 64 [106 - 22] s$^{-1}$, $k_{unz,RepE,100mV}$ = 0.12 [0.2 - 0.04] s$^{-1}$; 95 % CI; n = 5 and 7 respectively). Both oligonucleotides have a high melting temperature suggesting that at our lower working temperature (22 °C) both are fully hybridized (Figure 13). In agreement with previous reports,[29] we attribute the differences in unzipping kinetics to the differences in the first base pair to be unzipped. The RepE oligonucleotide begins with G-C while that of FOXM1 begins with A-T. Following the addition of FOXM1 protein, events showing an additional step are detected (Figure 3b), which correspond to the threading of protein-bound DNA molecules, detachment of the protein, unzipping and translocation of the DNA through the pore. Measurement of the fractions of bound and unbound DNA molecules allows us to calculate the $K_d$ = 0.98 $\pm$ 0.14 $\mu$M (mean $\pm$ SD, n=4) for the interaction (Figure 3c), which is in reasonable good agreement with previous reports using a DNA with multiple binding sites ($K_d$ = 0.22 $\pm$ 0.03, 2-3 binding sites).[37] The kinetics of protein release are fast and show weak dependence on voltage ($k_{off,80mV}$ = 43 [77 - 9] s$^{-1}$, $k_{off,90mV}$ = 57 [96 - 18] s$^{-1}$, and $k_{off,100mV}$ = 72 [117 - 27] s$^{-1}$; 95 % CI; n = 3, 5 and 5 respectively; Figure 14), suggesting that the applied force and the "peeling" mechanism are not relevant in this case. Nevertheless, here we work with the DNA-binding domain alone and it is possible that the full-length protein behaves differently.

To measure the inhibitory effect of FDI-6, we titrate a solution of FOXM1-DNA with the inhibitory compound (Figure 3d-f). In response to sequential additions of FDI-6, the fraction of events with a protein bound to DNA decreases, which implies an increase in the observed affinity ($K_{d,obs}$, Figure 3d-f). The data allow the determination of the affinity of the inhibitor for the protein ($K_{d,inhibitor}$) assuming a model where FDI-6 binding to FOXM1 completely prevents the protein-DNA interaction, there is only one site and FDI-6 binding is reversible (note that although the model may not be accurate it remains useful in practice). For the data displayed in Figure 3d-f, we obtain $K_{d,inhibitor}$ = 139 $\pm$ 73 $\mu$M (mean $\pm$ SD, n=7). Interestingly, more information can be gathered from our data. In the presence of FDI-6, DNA unzipping and translocation proceed with kinetics similar to those found when the compound is absent ($k_{unz,90mV}$ = 11 [18 - 4] s$^{-1}$, $k_{unz,90mV,FDI6}$ = 18 [30 - 6] s$^{-1}$; 95 % CI; n = 5 and 3 respectively). The kinetics of protein detachment from DNA are also similar in the presence of FDI-6 ($k_{off,90mV}$ = 57 [96 - 18] s$^{-1}$, $k_{off,90mV,FD16}$ = 49 [88 - 10] s$^{-1}$; 95 % CI; n = 5 and 3 respectively). The lack of effect on the kinetics of DNA unzipping and protein detachment suggests that the compound binds the protein (not the DNA), and that binding is competitive.

One of the major hurdles in the screening of protein-DNA interaction inhibitors is that a large fraction of active compounds interact non-specifically with the DNA. Furthermore, detecting this non-specific interaction requires a separate and laborious assay.[37] Because in our approach we detect the DNA unzipping and translocation, we tested whether we can also distinguish non-specific interactions that would otherwise be passed as false-positives. We used doxorubicin, a DNA intercalating agent that also interacts with the minor groove of dsDNA, as an example of a non-specific DNA binding molecule.[39] The interaction of doxorubicin with the DNA radically changed the ionic current signal, giving noisy long-lived events (Figure 9). This different behaviour allows easy discernment of the mechanism of action: if a potential inhibitor reduces the fraction of protein-bound complexes while maintaining the ionic current signature and the kinetics of DNA unzipping, it binds to the protein.

**Example 3-DNA-coded binding sites allows multiplexed analysis of an inhibitor.**

Nanopores produce different ionic current signals when different DNA sequences are threaded through them.[40] Motivated by this ability, we aimed to codify different target dsDNAs through the ssDNA extensions. According to our molecular model, the ionic current at levels P and D correspond to different segments of the ssDNA overhang threaded into the nanopore (Fig. 1a,b). We explored ssDNAs with different sequence patterns, and found that the residual current at level P depends on the nucleotide sequence of the last 20 bases of our ssDNA overhang (Figure 10, a finding in agreement with previous experiments on streptavidin-immobilized oligonucleotides.[41] This allowed us to codify one target DNA with 20 adenines at the 5' overhang and another one with 20 cytosines. The residual current at level D was mainly affected by the first base pair of the dsDNA because is in close contact with the constriction at the entrance of the pore.[42] This allowed us to codify one target DNA with a G-C base pair and the other with an A-T base pair (Figure 10). In addition to the residual current, the dwell time of level D can also be used to distinguish the identity of the molecule passing through the pore. Different base pairing at the unzipping point produce different unzipping times, with G-C unzipping slower than A-T and with the presence of non-hybridizing bases accelerating unzipping. We therefore used 3 parameters to classify the events: $I_{resD}$ (the residual current at level D), $I_{resP}$ (the residual current at level P) and $t_D$ (the dwell time at level D).

We used Reporter 1, which incorporates the binding sequence of RepE followed by an stretch of 11 adenines and a further stretch of 20 cytosines. Reporter 1 showed $I_{resD} = 0.189 \pm 0.008$ and $t_D = 0.25$ s$^{-1}$ when it was not bound to RepE, and $I_{resD} = 0.189 \pm 0.008$, $I_{resP} = 0.271 \pm 0.008$ and $t_D = 0.25$ s$^{-1}$ when RepE was bound (mean $\pm$ SD). Reporter 2 contained the binding sequence for FOXM1 followed by a stretch of 10 cytosines and then 20 adenines. Reporter 2 showed $I_{resD} = 0.219 \pm 0.005$ and $t_D = 66$ s$^{-1}$ when it was not bound to FOXM1 and $I_{resD} = 0.219 \pm 0.005$, $I_{resP} = 0.246 \pm 0.003$ and $t_D = 66$ s$^{-1}$ when FOXM1 was bound (mean $\pm$ SD, Fig. 4a,b and Figure 11). When all four species are present in solution, the expected 4 different signals are obtained. The analysis of each signal based on $I_{resP}$, $I_{resD}$ and $t_D$ allows the assignment of each event to one of the four possible species: free Reporter 1, RepE-bound Reporter 1, free Reporter 2 and FOXM1-bound Reporter 2. With this approach we obtain $K_d$ values for protein-DNA interactions similar to those obtained when the protein-DNA pairs were examined alone ($K_{d,FOXM1} = 0.75$ [1.03 - 0.56] $\mu$M, 95 % CI, in multiplexed measurements); $K_{d,FOXM1} = 0.98 \pm 0.14$ $\mu$M (alone); $K_{d,RepE} = 0.33$ [0.66 - 0.22] $\mu$M, 95 % CI, (in multiplexed measurements); $K_d = 0.34 \pm 0.08$ $\mu$M (alone), thereby validating the multiplexed approach (n = 4).

Having successfully shown the ability of the nanopore to measure the relative population of each member of the mixture, we determined whether the approach can detect the inhibition of FOXM1 binding caused by FDI-6, and its specificity (*i.e.* whether it also affects RepE or not) in the same experiment. We first measured the $K_d$ values of the two protein-DNA pairs present in a mixture of Reporter 1, Reporter 2, FOXM1 and RepE (we added smaller amounts of RepE and Reporter 1 as an internal control of our event classifier). Next, we added increasing amounts of FDI-6 and observed that the fraction of events assigned to free Reporter 2 (which contains the binding sequence for FOXM1) increased, while those of Reporter 2 with bound protein decreased. By contrast, the fractions of free and protein-bound Reporter 1 remained constant, indicating that the inhibitor FDI-6 had no effect on the interaction between RepE and its target sequence (Figure 4d).

**REFERENCES**

(1) Mylona, A., Theillet, F. X., Foster, C., Cheng, T. M., Miralles, F., Bates, P. A., Selenko, P., Treisman, R. Opposing Effects of Elk-1 Multisite Phosphorylation Shape its Response to ERK Activation. Science 2016, 354, 233-237.

(2) Nakamura, A., Wada, C., Miki, K. Structural Basis for Regulation of Bifunctional Roles in Replication Initiator Protein. Proc Natl Acad Sci U S A. 2007, 104, 18484-18489.

(3) Ishiai, M., Wada, C., Kawasaki, Y., Yura, T. Replication Initiator Protein RepE of Mini-F Plasmid: Functional Differentiation Between Monomers (Initiator) and Dimers (Autogenous Repressor). Proc Natl Acad Sci U S A. 1994, 91, 3839-3843.

(4) Philips, S.J., Canalizo-Hernandez, M., Yildirim, I., Schatz, G. C., Mondragón, A., O'Halloran, T. V. TRANSCRIPTION. Allosteric Transcriptional Regulation via Changes in the Overall Topology of the Core Promoter. Science 2015, 349, 877-881.

(5) He, Q., Johnston, J., Zeitlinger, J. ChIP-Nexus Enables Improved Detection of In Vivo Transcription Factor Binding Footprints. Nat Biotechnol. 2015, 33, 395-401.

(6) Jolma, A., Yin, Y., Nitta, K. R., Dave, K., Popov, A., Taipale, M., Enge, M., Kivioja, T., Morgunova, E., Taipale, J. DNA-Dependent Formation of Transcription Factor Pairs Alters their Binding Specificity. Nature 2015, 527, 384-388.

(7) Nutiu, R., Friedman, R. C., Luo, S., Khrebtukova, I., Silva, D., Li, R., Zhang, L., Schroth, G. P., Burge, C. B. Direct Measurement of DNA Affinity Landscapes on a High-Throughput Sequencing Instrument.. Nat Biotechnol. 2011, 29, 659-664.

(8) van Oijen, A.M. Single-Molecule Approaches to Characterizing Kinetics of Biomolecular Interactions. Curr Opin Biotechnol. 2011, 22, 75-80.

(9) Blosser, T.R., Loeff, L., Westra, E. R., Vlot, M., Künne, T., Sobota, M., Dekker, C., Brouns, S. J., Joo, C. Two Distinct DNA Binding Modes Guide Dual Roles of a CRISPR-Cas Protein Complex. Mol Cell. 2015, 58, 60-70.

(10) Forget, A.L., Dombrowski, C.C., Amitani, I., Kowalczykowski, S.C. Exploring Protein-DNA Interactions in 3D Using In Situ Construction, Manipulation and Visualization of Individual DNA Dumbbells with Optical Traps, Microfluidics and Fluorescence Microscopy. Nat Protoc. 2013, 8, 525-538.

(11) Maglia, G., Heron, A.J., Stoddart, D., Japrung, D., Bayley, H. Analysis of Single Nucleic Acid Molecules with Protein Nanopores. Methods Enzymol. 2010, 475, 591-623.

(12) Mathé, J., Visram, H., Viasnoff, V., Rabin, Y., Meller, A. Nanopore Unzipping of Individual DNA Hairpin Molecules. Biophys J. 2004, 87, 3205-3212.

(13) Nivala, J., Marks, D.B., Akeson, M. Unfoldase-Mediated Protein Translocation through an A-Hemolysin Nanopore. Nat Biotechnol. 2013, 31, 247-250.

(14) Rodriguez-Larrea, D., Bayley, H. Multistep Protein Unfolding during Nanopore Translocation. Nat Nanotechnol. 2013, 8, 288-295.

(15) Rodriguez-Larrea, D., Bayley, H. Protein Co-Translocational Unfolding Depends on the Direction of Pulling. Nat Commun. 2014, 5, 4841.

(16) Lee, J., Boersma, A. J., Boudreau, M. A., Cheley, S., Daltrop, O., Li, J., Tamagaki, H., Bayley, H. Semisynthetic Nanoreactor for Reversible Single-Molecule Covalent Chemistry. ACS Nano. 2016, 10, 8843-8850.

(17) Hayden, E.C. Pint-Sized DNA Sequencer Impresses First Users. Nature 2015, 521, 15-16.

(18) Benner, S., Chen, R. J., Wilson, N. A., Abu-Shumays, R., Hurt, N., Lieberman, K. R., Deamer, D. W., Dunbar, W. B., Akeson, M. Sequence-Specific Detection of Individual DNA Polymerase Complexes in Real Time Using a Nanopore. Nat Nanotechnol. 2007, 2, 718-724.

(19) Hornblower, B., Coombs, A., Whitaker, R. D., Kolomeisky, A., Picone, S. J., Meller, A., Akeson, M. Single-Molecule Analysis of DNA-Protein Complexes Using Nanopores. Nat Methods 2007, 4, 315-317.

(20) Van Meervelt, V., Soskine, M., Maglia, G. Detection of Two Isomeric Binding Configurations in a Protein-Aptamer Complex with a Biological Nanopore. ACS Nano. 2014, 8, 12826-12835.

(21) Harrington, L., Alexander, L.T., Knapp, S., Bayley, H. Pim Kinase Inhibitors Evaluated with a Single-Molecule Engineered Nanopore Sensor. Angew Chem Int Ed Engl. 2015, 54, 8154-8159.

(22) Bell, N.A., Keyser, U.F. Specific Protein Detection Using Designed DNA Carriers and Nanopores. J Am Chem Soc. 2015, 137, 2035-2041.

(23) Smeets, R.M., Kowalczyk, S.W., Hall, A.R., Dekker, N.H., Dekker, C. Translocation of RecA-Coated Double-Stranded DNA through Solid-State Nanopores. Nano Lett. 2009, 9, 3089-3096.

(24) Ivankin, A., Carson, S., Kinney, S.R., Wanunu, M. Fast, Label-Free Force Spectroscopy of Histone-DNA Interactions in Individual Nucleosomes Using Nanopores. J Am Chem Soc. 2013, 135, 15350-15352.

(25) Squires, A., Atas, E., Meller, A. Nanopore Sensing of Individual Transcription Factors Bound to DNA. Sci Rep. 2015, 5, 11643.

(26) Wanunu, M., Sutin, J., Meller, A. DNA Profiling Using Solid-State Nanopores: Detection of DNA-Binding Molecules. Nano Lett. 2009, 9, 3498-3502.

(27) Bell, N.A., Keyser, U.F. Digitally Encoded DNA Nanostructures for Multiplexed, Single-Molecule Protein Sensing with Nanopores. Nat Nanotechnol. 2016, 11, 645-651.

(28) Kasianowicz, J.J., Henrickson, S.E., Weetall, H.H., Robertson, B. Simultaneous Multianalyte Detection with a Nanometer-Scale Pore. Anal. Chem. 2001, 73, 2268-2272.

(29) Renner, S., Bessonov, A., Gerland, U., Simmel, F.C. Sequence-Dependent Unfolding Kinetics of DNA Hairpins Studied by Nanopore Force Spectroscopy. J Phys Condens Matter. 2010, 22, 454119.

(30) Stoddart, D., Franceschini, L., Heron, A., Bayley, H., Maglia, G. DNA Stretching and Optimization of Nucleobase Recognition in Enzymatic Nanopore Sequencing. Nanotechnology 2015, 26, 084002.

(31) Maillard, R.A., Christol, G., Sen, M., Righini, M., Tan, J., Kaiser, C. M., Hodges, C., Martin, A., Bustamante, C. ClpX(P) Generates Mechanical Force to Unfold and Translocate its Protein Substrates. Cell 2011, 145, 459-469.

(32) Komori, H., Matsunaga, F, Higuchi, Y, Ishiai, M., Wada, C., Miki, K. Crystal Structure of a Prokaryotic Replication Initiator Protein Bound to DNA at 2.6 A Resolution. EMBO J. 1999, 18, 4597-4607.

(33) Liu, B., Baskin, R.J., Kowalczykowski, S.C. DNA Unwinding Heterogeneity by RecBCD Results from Static Molecules Able to Equilibrate. Nature 2013, 500, 482-485.

(34) Koch, S.J., Shundrovsky, A., Jantzen, B.C., Wang, M.D. Probing Protein-DNA Interactions by Unzipping a Single DNA Double Helix. Biophys J. 2002, 83, 1098-1105.

(35) Singleton, M.R., Dillingham, M.S., Wigley, D.B. Structure and Mechanism of Helicases and Nucleic Acid Translocases. Annu Rev Biochem. 2007, 76, 23-50.

(36) Fairman, M.E., Maroney, P. A., Wang, W., Bowers, H. A., Gollnick, P., Nilsen, T. W., Jankowsky, E. Protein Displacement by DExH/D "RNA helicases" without Duplex Unwinding. Science 2004, 304, 730-734.

(37) Gormally, M.V., Dexheimer, T. S., Marsico, G., Sanders, D. A., Lowe, C., Matak-Vinkovic, D., Michael, S., Jadhav, A., Rai, G., Maloney, D. J., Simeonov, A., Balasubramanian, S. Suppression of the FOXM1 Transcriptional Programme via Novel Small Molecule Inhibition. Nat Commun. 2014, 5, 5165.

(38) Littler, D.R., Alvarez-Fernández, M., Stein, A., Hibbert, R. G., Heidebrecht, T., Aloy, P., Medema, R. H., Perrakis, A. Structure of the FoxM1 DNA-Recognition Domain Bound to a Promoter Sequence. Nucleic Acids Res. 2010, 38, 4527-4538.

(39) Welch, J.J., Rauscher, F.J., 3rd, Beerman, T.A. Targeting DNA-binding Drugs to Sequence-Specific Transcription Factor.DNA Complexes. Differential Effects of Intercalating and Minor Groove Binding Drugs. J Biol Chem. 1994, 269, 31051-31058.

(40) Meller, A., Nivon, L., Brandin, E., Golovchenko, J., Branton, D. Rapid Nanopore Discrimination between Single Oligonucleotide Molecules. Proc. Natl. Acad. Sci. U.S.A. 2000, 97, 1079-1084.

(41) Stoddart, D., Heron, A.J., Mikhailova, E., Maglia, G., Bayley H. Single-Nucleotide Discrimination in Immobilized DNA Oligonucleotides with a Biological Nanopore. Proc Natl Acad Sci U S A. 2009, 106, 7702-7707.

(42) Vercoutere, W.A., Winters-Hilt, S., DeGuzman, V. S., Deamer, D., Ridino, S. E., Rodgers, J. T., Olsen, H. E., Marziali, A., Akeson, M. Discrimination among Individual Watson-Crick Base Pairs at the Termini of Single DNA Hairpin Molecules. Nucleic Acids Res. 2003, 31, 1311-1318.

(43) Lazo, J. S., Sharlow, E. Drugging Undruggable Molecular Cancer Targets. Ann. Rev. Pharmacol Toxicol 2016, 56, 23-40.

(44) Eder, J., Sedrani, R., Wiesmann, C. The Discovery of First-in-Class Drugs: Origins and Evolution. Nat Rev Drug Discov. 2014, 13, 577-587.

(45) Baaken, G., Sondermann, M., Schlemmer, C., Rühe, J., Behrends, J. C. Planar Microelectrode-Cavity Array for High-Resolution and Parallel Electrical Recording of Membrane Ionic Currents. Lab Chip 2008, 6, 938-944.

(46) Walker, B., Krishnasastry, M., Zorn, L., Kasianowicz, J., Bayley, H. Functional Expression of the A-Hemolysin of Staphylococcus Aureus in Intact Escherichia Coli and in Cell Lysates. J Biol Chem. 1992, 267, 10902-10908.

(47) Pettersen, E.F., Goddard, T. D., Huang, C. C., Couch, G. S., Greenblatt, D. M., Meng, E. C., Ferrin, T. E. UCSF Chimera - A Visualization System for Exploratory Research and Analysis. J. Comput. Chem. 2004, 25, 1605-1612.

**Claims**

1.  Method for detecting the interaction between a protein and a DNA target sequence which comprises:

    a) putting into contact the protein with a DNA molecule, said DNA molecule comprising a ssDNA region and a dsDNA region, wherein the dsDNA region contains the DNA target sequence and with a planar lipid bilayer containing at least one alpha hemolysin nanopore under conditions allowing the interaction between the protein, the DNA molecule and the planar lipid bilayer,
    b) applying a positive electric potential across the lipid bilayer which is capable of electrophoretically driving the ssDNA through the alpha-hemolysin nanopore within the lipid bilayer, and

c) measuring the ionic current through the alpha-hemolysin nanopores,

wherein if an initial level in the ionic current signal having higher conductance is detected, is indicative that there is protein-DNA interaction.

2. Method for identifying a compound capable of modulating the interaction between a protein and a DNA target sequence which comprises:

a) putting into contact the compound with the protein, a DNA molecule, said DNA molecule comprising a ssDNA region and a dsDNA region, wherein the dsDNA region contains the DNA target sequence and with a planar lipid bilayer containing at least one alpha hemolysin nanopore, under conditions allowing the interaction between the compound, the protein, the DNA molecule and the planar lipid bilayer,
b) applying a positive electric potential across the lipid bilayer which is capable of electrophoretically driving the ssDNA through the alfa hemolysin nanopore within the lipid bilayer, and
c) measuring the ionic current through the alpha-hemolysin nanopores,

wherein if an initial level in the ionic current signal having higher conductance is detected in comparison with the signal in the absence of said compound, is indicative the compound is an enhancer of the protein-DNA interaction, and wherein if an initial level in the ionic current signal having higher conductance is not detected in comparison with the signal in the absence of said compound, is indicative the compound is an inhibitor of the protein-DNA interaction.

3. Method according to any of claims 1 or 2, wherein the DNA molecule comprises a self-complementary 3' region which hybridizes to form a hairpin region and a 5' region comprising the ssDNA.

4. Method according to claim 3wherein the hairpin region has a length of at least 10 to 36 bp, preferably 21 bp.

5. Method according to any of claim 3 or 4 wherein the 5'ssDNA region has a length of at least 20 to 100 nucleotides, preferably 30 nucleotides.

6. Method according to any of claim 1 to 5 wherein the alpha hemolysin pore within the lipid bilayer has an average internal diameter of the pore of about 1.3 nm.

7. Method according to any of claims 1 to 6 wherein the dsDNA sequence has a high melting temperature.

8. The method according to any of claims 1 to 7 wherein the protein is a transcription factor.

9. The method according to any of claims 1 to 8 wherein the lipid bilayer is formed by diphytanoyl-sn-glycero-3-phosphocholine.

10. A kit comprising at least one protein, at least one DNA molecule, said DNA molecule comprising a ssDNA region and a dsDNA region, wherein the dsDNA region contains a DNA target sequence, and a planar lipid bilayer containing at least one alpha hemolysin nanopore.

11. Kit according to claim 10, wherein the DNA molecule comprises a self-complementary 3' region which hybridizes to form a hairpin region comprising the target sequence and a 5' region comprising the ssDNA.

12. Kit according to claim 11 wherein the hairpin DNA has a length of at least 10 to 36 bp, preferably 21 bp.

13. Kit according to any of claims 10 to 12 wherein the 5'ssDNA region has a length of at least 20 to 100 nucleotides, preferably 30 nucleotides.

14. Kit according to any of claim 10 to 13 wherein the alfa hemolysin pore has an internal diameter of the pore of 1.3 nm.

15. Use of the kit according to any of claims 10 to 14 for detecting protein-DNA interaction and/or for identifying a compound capable of modulating protein-DNA interaction.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

## EUROPEAN SEARCH REPORT

Application Number

EP 17 38 2287

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SEICO BENNER ET AL: "Sequence-specific detection of individual DNA polymerase complexes in real time using a nanopore", NATURE NANOTECHNOLOGY, vol. 2, no. 11, 1 November 2007 (2007-11-01), pages 718-724, XP055055941, ISSN: 1748-3387, DOI: 10.1038/nnano.2007.344 * page 718, column 1, bottom - column 2; page 720 * | 1-15 | INV. C12Q1/68 |
| X | WO 2008/124107 A1 (UNIV CALIFORNIA [US]; AKESON MARK A [US]; DEAMER DAVID W [US]; DUNBAR) 16 October 2008 (2008-10-16) * claims and figures * | 1-15 | |
| X | WO 2012/033524 A2 (UNIV CALIFORNIA [US]; CHERF GERALD MAXWELL [US]; LIBERMAN KATE R [US];) 15 March 2012 (2012-03-15) * examples XIII-XV; claims; figures * | 1-7,9-15 | |
| X | EP 2 886 663 A1 (CENTRE NAT RECH SCIENT [FR]; ECOLE NORMALE SUPÉRIEURE [FR]; UNIV D EVR) 24 June 2015 (2015-06-24) * column 19, bottom; claims 1-16; figures * | 1,10,15 | **TECHNICAL FIELDS SEARCHED (IPC)** <br> C12Q |
| X | WO 2016/053891 A1 (UNIV CALIFORNIA [US]) 7 April 2016 (2016-04-07) * figures, claims 9-22; page 8, lines 20-27 * | 1,10,15 | |
| X | WO 2010/117470 A2 (PACIFIC BIOSCIENCES CALIFORNIA [US]; TURNER STEPHEN [US]; FLUSBERG BEN) 14 October 2010 (2010-10-14) * claims; figures * | 1,10,15 | |

-/--

The present search report has been drawn up for all claims

| Place of search | Date of completion of the search | Examiner |
|---|---|---|
| Munich | 27 July 2017 | Hennard, Christophe |

EPO FORM 1503 03.82 (P04C01)

page 1 of 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | A.H. Squires ET AL: "Single-Molecule Characterization of DNA-Protein Interactions Using Nanopore Biosensors" In: "METHODS IN ENZYMOLOGY", 1 January 2017 (2017-01-01), ACADEMIC PRESS, US, XP055394114, ISSN: 0076-6879 vol. 582, pages 353-385, DOI: 10.1016/bs.mie.2016.08.010, * figure 1; figure 3D * ----- | 1-15 | |
| Y | HORNBLOWER B ET AL: "Single-molecule analysis of DNA-protein complexes using nanopores", NATURE MET,, vol. 4, no. 4, 1 January 2007 (2007-01-01) , pages 315-317, XP009164677, ISSN: 1548-7091, DOI: 10.1038/NMETH1021 * abstract; page 315, column 2; figure 1 * ----- | 1-15 | |

TECHNICAL FIELDS
SEARCHED      (IPC)

The present search report has been drawn up for all claims

| Place of search | Date of completion of the search | Examiner |
|---|---|---|
| Munich | 27 July 2017 | Hennard, Christophe |

EPO FORM 1503 03.82 (P04C01)

page 2 of 2

## ANNEX TO THE EUROPEAN SEARCH REPORT
## ON EUROPEAN PATENT APPLICATION NO.

EP 17 38 2287

This annex lists the patent family members relating to the patent documents cited in the above-mentioned European search report.
The members are as contained in the European Patent Office EDP file on
The European Patent Office is in no way liable for these particulars which are merely given for the purpose of information.

27-07-2017

| Patent document cited in search report | | | Publication date | Patent family member(s) | | | Publication date |
|---|---|---|---|---|---|---|---|
| WO 2008124107 | A1 | | 16-10-2008 | AU | 2008236694 | A1 | 16-10-2008 |
| | | | | CA | 2684801 | A1 | 16-10-2008 |
| | | | | CN | 101680873 | A | 24-03-2010 |
| | | | | EP | 2156179 | A1 | 24-02-2010 |
| | | | | JP | 5514361 | B2 | 04-06-2014 |
| | | | | JP | 5646987 | B2 | 24-12-2014 |
| | | | | JP | 5956535 | B2 | 27-07-2016 |
| | | | | JP | 2010524436 | A | 22-07-2010 |
| | | | | JP | 2014060997 | A | 10-04-2014 |
| | | | | JP | 2015051013 | A | 19-03-2015 |
| | | | | KR | 20100031498 | A | 22-03-2010 |
| | | | | US | 2010035260 | A1 | 11-02-2010 |
| | | | | US | 2011005918 | A1 | 13-01-2011 |
| | | | | US | 2011174625 | A1 | 21-07-2011 |
| | | | | US | 2013118902 | A1 | 16-05-2013 |
| | | | | US | 2014034517 | A1 | 06-02-2014 |
| | | | | US | 2014255918 | A1 | 11-09-2014 |
| | | | | US | 2014346059 | A1 | 27-11-2014 |
| | | | | US | 2016040230 | A1 | 11-02-2016 |
| | | | | US | 2016209350 | A1 | 21-07-2016 |
| | | | | US | 2016289758 | A1 | 06-10-2016 |
| | | | | WO | 2008124107 | A1 | 16-10-2008 |
| WO 2012033524 | A2 | | 15-03-2012 | CN | 103392008 | A | 13-11-2013 |
| | | | | EP | 2614156 | A2 | 17-07-2013 |
| | | | | US | 2014051068 | A1 | 20-02-2014 |
| | | | | WO | 2012033524 | A2 | 15-03-2012 |
| EP 2886663 | A1 | | 24-06-2015 | EP | 2886663 | A1 | 24-06-2015 |
| | | | | EP | 3083990 | A1 | 26-10-2016 |
| | | | | JP | 2017500046 | A | 05-01-2017 |
| | | | | US | 2016319344 | A1 | 03-11-2016 |
| | | | | WO | 2015092039 | A1 | 25-06-2015 |
| WO 2016053891 | A1 | | 07-04-2016 | NONE | | | |
| WO 2010117470 | A2 | | 14-10-2010 | US | 2010331194 | A1 | 30-12-2010 |
| | | | | US | 2014061048 | A1 | 06-03-2014 |
| | | | | US | 2015159213 | A1 | 11-06-2015 |
| | | | | US | 2017122929 | A1 | 04-05-2017 |
| | | | | US | 2017168040 | A1 | 15-06-2017 |
| | | | | WO | 2010117470 | A2 | 14-10-2010 |

EPO FORM P0459

For more details about this annex : see Official Journal of the European Patent Office, No. 12/82

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MYLONA, A. ; THEILLET, F. X. ; FOSTER, C. ; CHENG, T. M. ; MIRALLES, F. ; BATES, P. A. ; SELENKO, P. ; TREISMAN, R.** Opposing Effects of Elk-1 Multisite Phosphorylation Shape its Response to ERK Activation. *Science,* 2016, vol. 354, 233-237
- **NAKAMURA, A. ; WADA, C. ; MIKI, K.** Structural Basis for Regulation of Bifunctional Roles in Replication Initiator Protein. *Proc Natl Acad Sci U S A.,* 2007, vol. 104, 18484-18489
- **ISHIAI, M. ; WADA, C. ; KAWASAKI, Y. ; YURA, T.** Replication Initiator Protein RepE of Mini-F Plasmid: Functional Differentiation Between Monomers (Initiator) and Dimers (Autogenous Repressor). *Proc Natl Acad Sci U S A.,* 1994, vol. 91, 3839-3843
- **PHILIPS, S.J. ; CANALIZO-HERNANDEZ, M. ; YILDIRIM, I. ; SCHATZ, G. C. ; MONDRAGÓN, A. ; O'HALLORAN, T. V.** TRANSCRIPTION. Allosteric Transcriptional Regulation via Changes in the Overall Topology of the Core Promoter. *Science,* 2015, vol. 349, 877-881
- **HE, Q. ; JOHNSTON, J. ; ZEITLINGER, J.** ChIP-Nexus Enables Improved Detection of In Vivo Transcription Factor Binding Footprints. *Nat Biotechnol.,* 2015, vol. 33, 395-401
- **JOLMA, A. ; YIN, Y. ; NITTA, K. R. ; DAVE, K. ; POPOV, A. ; TAIPALE, M. ; ENGE, M. ; KIVIOJA, T. ; MORGUNOVA, E. ; TAIPALE, J.** DNA-Dependent Formation of Transcription Factor Pairs Alters their Binding Specificity. *Nature,* 2015, vol. 527, 384-388
- **NUTIU, R. ; FRIEDMAN, R. C. ; LUO, S. ; KHREBTUKOVA, I. ; SILVA, D. ; LI, R. ; ZHANG, L. ; SCHROTH, G. P. ; BURGE, C. B.** Direct Measurement of DNA Affinity Landscapes on a High-Throughput Sequencing Instrument. *Nat Biotechnol.,* 2011, vol. 29, 659-664
- **VAN OIJEN, A.M.** Single-Molecule Approaches to Characterizing Kinetics of Biomolecular Interactions. *Curr Opin Biotechnol.,* 2011, vol. 22, 75-80
- **BLOSSER, T.R. ; LOEFF, L. ; WESTRA, E. R. ; VLOT, M. ; KÜNNE, T. ; SOBOTA, M. ; DEKKER, C. ; BROUNS, S. J. ; JOO, C.** Two Distinct DNA Binding Modes Guide Dual Roles of a CRISPR-Cas Protein Complex. *Mol Cell,* 2015, vol. 58, 60-70

- **FORGET, A.L. ; DOMBROWSKI, C.C. ; AMITANI, I. ; KOWALCZYKOWSKI, S.C.** Exploring Protein-DNA Interactions in 3D Using In Situ Construction, Manipulation and Visualization of Individual DNA Dumbbells with Optical Traps, Microfluidics and Fluorescence Microscopy. *Nat Protoc.,* 2013, vol. 8, 525-538
- **MAGLIA, G. ; HERON, A.J. ; STODDART, D. ; JAPRUNG, D. ; BAYLEY, H.** Analysis of Single Nucleic Acid Molecules with Protein Nanopores. *Methods Enzymol.,* 2010, vol. 475, 591-623
- **MATHÉ, J. ; VISRAM, H. ; VIASNOFF, V. ; RABIN, Y. ; MELLER, A.** Nanopore Unzipping of Individual DNA Hairpin Molecules. *Biophys J.,* 2004, vol. 87, 3205-3212
- **NIVALA, J. ; MARKS, D.B. ; AKESON, M.** Unfoldase-Mediated Protein Translocation through an A-Hemolysin Nanopore. *Nat Biotechnol.,* 2013, vol. 31, 247-250
- **RODRIGUEZ-LARREA, D. ; BAYLEY, H.** Multistep Protein Unfolding during Nanopore Translocation. *Nat Nanotechnol.,* 2013, vol. 8, 288-295
- **RODRIGUEZ-LARREA, D. ; BAYLEY, H.** Protein Co-Translocational Unfolding Depends on the Direction of Pulling. *Nat Commun.,* 2014, vol. 5, 4841
- **LEE, J. ; BOERSMA, A. J. ; BOUDREAU, M. A. ; CHELEY, S. ; DALTROP, O. ; LI, J. ; TAMAGAKI, H. ; BAYLEY, H.** Semisynthetic Nanoreactor for Reversible Single-Molecule Covalent Chemistry. ACS Nano, 2016, vol. 10, 8843-8850
- **HAYDEN, E.C.** Pint-Sized DNA Sequencer Impresses First Users. *Nature,* 2015, vol. 521, 15-16
- **BENNER, S. ; CHEN, R. J. ; WILSON, N. A. ; ABU-SHUMAYS, R. ; HURT, N. ; LIEBERMAN, K. R. ; DEAMER, D. W. ; DUNBAR, W. B. ; AKESON, M.** Sequence-Specific Detection of Individual DNA Polymerase Complexes in Real Time Using a Nanopore. *Nat Nanotechnol.,* 2007, vol. 2, 718-724
- **HORNBLOWER, B. ; COOMBS, A. ; WHITAKER, R. D. ; KOLOMEISKY, A. ; PICONE, S. J. ; MELLER, A. ; AKESON, M.** Single-Molecule Analysis of DNA-Protein Complexes Using Nanopores. *Nat Methods,* 2007, vol. 4, 315-317
- **VAN MEERVELT, V. ; SOSKINE, M. ; MAGLIA, G.** Detection of Two Isomeric Binding Configurations in a Protein-Aptamer Complex with a Biological Nanopore. ACS Nano, 2014, vol. 8, 12826-12835

- **HARRINGTON, L. ; ALEXANDER, L.T. ; KNAPP, S. ; BAYLEY, H.** Pim Kinase Inhibitors Evaluated with a Single-Molecule Engineered Nanopore Sensor. *Angew Chem Int Ed Engl.,* 2015, vol. 54, 8154-8159
- **BELL, N.A. ; KEYSER, U.F.** Specific Protein Detection Using Designed DNA Carriers and Nanopores. *J Am Chem Soc.,* 2015, vol. 137, 2035-2041
- **SMEETS, R.M. ; KOWALCZYK, S.W. ; HALL, A.R. ; DEKKER, N.H. ; DEKKER, C.** Translocation of ReсA-Coated Double-Stranded DNA through Solid-State Nanopores. *Nano Lett.,* 2009, vol. 9, 3089-3096
- **IVANKIN, A. ; CARSON, S. ; KINNEY, S.R. ; WANUNU, M.** Fast, Label-Free Force Spectroscopy of Histone-DNA Interactions in Individual Nucleosomes Using Nanopores. *J Am Chem Soc.,* 2013, vol. 135, 15350-15352
- **SQUIRES, A. ; ATAS, E. ; MELLER, A.** Nanopore Sensing of Individual Transcription Factors Bound to DNA. *Sci Rep.,* 2015, vol. 5, 11643
- **WANUNU, M. ; SUTIN, J. ; MELLER, A.** DNA Profiling Using Solid-State Nanopores: Detection of DNA-Binding Molecules. *Nano Lett.,* 2009, vol. 9, 3498-3502
- **BELL, N.A. ; KEYSER, U.F.** Digitally Encoded DNA Nanostructures for Multiplexed, Single-Molecule Protein Sensing with Nanopores. *Nat Nanotechnol.,* 2016, vol. 11, 645-651
- **KASIANOWICZ, J.J. ; HENRICKSON, S.E. ; WEETALL, H.H. ; ROBERTSON, B.** Simultaneous Multianalyte Detection with a Nanometer-Scale Pore. *Anal. Chem.,* 2001, vol. 73, 2268-2272
- **RENNER, S. ; BESSONOV, A. ; GERLAND, U. ; SIMMEL, F.C.** Sequence-Dependent Unfolding Kinetics of DNA Hairpins Studied by Nanopore Force Spectroscopy. *J Phys Condens Matter,* 2010, vol. 22, 454119
- **STODDART, D. ; FRANCESCHINI, L. ; HERON, A. ; BAYLEY, H. ; MAGLIA, G.** DNA Stretching and Optimization of Nucleobase Recognition in Enzymatic Nanopore Sequencing. *Nanotechnology,* 2015, vol. 26, 084002
- **MAILLARD, R.A. ; CHRISTOL, G. ; SEN, M. ; RIGHINI, M. ; TAN, J. ; KAISER, C. M. ; HODGES, C. ; MARTIN, A. ; BUSTAMANTE, C.** ClpX(P) Generates Mechanical Force to Unfold and Translocate its Protein Substrates. *Cell,* 2011, vol. 145, 459-469
- **KOMORI, H. ; MATSUNAGA, F ; HIGUCHI, Y ; ISHI-AI, M. ; WADA, C. ; MIKI, K.** Crystal Structure of a Prokaryotic Replication Initiator Protein Bound to DNA at 2.6 A Resolution. *EMBO J.,* 1999, vol. 18, 4597-4607
- **LIU, B. ; BASKIN, R.J. ; KOWALCZYKOWSKI, S.C.** DNA Unwinding Heterogeneity by RecBCD Results from Static Molecules Able to Equilibrate. *Nature,* 2013, vol. 500, 482-485
- **KOCH, S.J. ; SHUNDROVSKY, A. ; JANTZEN, B.C. ; WANG, M.D.** Probing Protein-DNA Interactions by Unzipping a Single DNA Double Helix. *Biophys J.,* 2002, vol. 83, 1098-1105
- **SINGLETON, M.R. ; DILLINGHAM, M.S. ; WIGLEY, D.B.** Structure and Mechanism of Helicases and Nucleic Acid Translocases. *Annu Rev Biochem.,* 2007, vol. 76, 23-50
- **FAIRMAN, M.E. ; MARONEY, P. A. ; WANG, W. ; BOWERS, H. A. ; GOLLNICK, P. ; NILSEN, T. W. ; JANKOWSKY, E.** Protein Displacement by DExH/D "RNA helicases" without Duplex Unwinding. *Science,* 2004, vol. 304, 730-734
- **GORMALLY, M.V. ; DEXHEIMER, T. S. ; MARSICO, G. ; SANDERS, D. A. ; LOWE, C. ; MATAK-VINKOVIC, D. ; MICHAEL, S. ; JADHAV, A. ; RAI, G. ; MALONEY, D. J.** Suppression of the FOXM1 Transcriptional Programme via Novel Small Molecule Inhibition. *Nat Commun.,* 2014, vol. 5, 5165
- **LITTLER, D.R. ; ALVAREZ-FERNÁNDEZ, M. ; STEIN, A. ; HIBBERT, R. G. ; HEIDEBRECHT, T. ; ALOY, P. ; MEDEMA, R. H. ; PERRAKIS, A.** Structure of the FoxM1 DNA-Recognition Domain Bound to a Promoter Sequence. *Nucleic Acids Res.,* 2010, vol. 38, 4527-4538
- **WELCH, J.J. ; RAUSCHER, F.J. ; BEERMAN, T.A.** Targeting DNA-binding Drugs to Sequence-Specific Transcription Factor.DNA Complexes. Differential Effects of Intercalating and Minor Groove Binding Drugs. *J Biol Chem.,* 1994, vol. 269, 31051-31058
- **MELLER, A. ; NIVON, L. ; BRANDIN, E. ; GOLOVCHENKO, J. ; BRANTON, D.** Rapid Nanopore Discrimination between Single Oligonucleotide Molecules. *Proc. Natl. Acad. Sci. U.S.A.,* 2000, vol. 97, 1079-1084
- **STODDART, D. ; HERON, A.J. ; MIKHAILOVA, E. ; MAGLIA, G. ; BAYLEY H.** Single-Nucleotide Discrimination in Immobilized DNA Oligonucleotides with a Biological Nanopore. *Proc Natl Acad Sci U S A.,* 2009, vol. 106, 7702-7707
- **VERCOUTERE, W.A. ; WINTERS-HILT, S. ; DEGUZMAN, V. S. ; DEAMER, D. ; RIDINO, S. E. ; RODGERS, J. T. ; OLSEN, H. E. ; MARZIALI, A. ; AKESON, M.** Discrimination among Individual Watson-Crick Base Pairs at the Termini of Single DNA Hairpin Molecules. *Nucleic Acids Res.,* 2003, vol. 31, 1311-1318
- **LAZO, J. S. ; SHARLOW, E.** Drugging Undruggable Molecular Cancer Targets. *Ann. Rev. Pharmacol Toxicol,* 2016, vol. 56, 23-40
- **EDER, J. ; SEDRANI, R. ; WIESMANN, C.** The Discovery of First-in-Class Drugs: Origins and Evolution. *Nat Rev Drug Discov.,* 2014, vol. 13, 577-587

- **BAAKEN, G. ; SONDERMANN, M. ; SCHLEMMER, C. ; RÜHE, J. ; BEHRENDS, J. C.** Planar Microelectrode-Cavity Array for High-Resolution and Parallel Electrical Recording of Membrane Ionic Currents. *Lab Chip,* 2008, vol. 6, 938-944
- **WALKER, B. ; KRISHNASASTRY, M. ; ZORN, L. ; KASIANOWICZ, J. ; BAYLEY, H.** Functional Expression of the A-Hemolysin of Staphylococcus Aureus in Intact Escherichia Coli and in Cell Lysates. *J Biol Chem.,* 1992, vol. 267, 10902-10908
- **PETTERSEN, E.F. ; GODDARD, T. D. ; HUANG, C. C. ; COUCH, G. S. ; GREENBLATT, D. M. ; MENG, E. C. ; FERRIN, T. E.** UCSF Chimera - A Visualization System for Exploratory Research and Analysis. *J. Comput. Chem.,* 2004, vol. 25, 1605-1612

SEQUENCE LISTING

<110> Universidad del País Vasco

<120> Method for detecting protein-DNA interaction

<130> P14699EP00

<160> 7

<170> PatentIn version 3.5

<210> 1
<211> 20
<212> PRT
<213> Artifical sequence

<400> 1

Met Ala His His His His His His Ser Ala Ala Leu Glu Val Leu Phe
1               5                   10                  15


Gln Gly Pro Gly
            20


<210> 2
<211> 78
<212> DNA
<213> Artificial sequence

<220>
<223> RepE (DIRECT)

<400> 2
aaaaaaaaaa aaaaaaaaaa cccccccccc cctgtgacaa attgccctca gcacacactg      60

agggcaattt gtcacagg                                                   78


<210> 3
<211> 78
<212> DNA
<213> Artificial sequence

<220>
<223> RepE (INVERSE)

<400> 3
cccccccccc cccccccccc aaaaaaaaaa actgtgacaa attgccctca gcacacactg      60

agggcaattt gtcacagg                                                   78


<210> 4
<211> 78
<212> DNA
<213> Artificial sequence

<220>
<223> FOXM1

```
<400>  4
aaaaaaaaaa aaaaaaaaaa cccccccccc aaattgttta taaacagccc gcacacacgg        60

gctgtttata aacaattt                                                      78


<210>  5
<211>  74
<212>  DNA
<213>  Artificial sequence

<220>
<223>  RepE (LevelP).Between the nucleotide in position 20 and the
       nucleotide in position 21. there is an spacer molecule of
       hexaethylene glycol

<400>  5
aaaaaaaaaa aaaaaaaaaa ccccccccct gtgacaaatt gccctcagca cacactgagg        60

gcaatttgtc acag                                                          74


<210>  6
<211>  78
<212>  DNA
<213>  Artificial sequence

<220>
<223>  Mismatched

<400>  6
aaaaaaaaaa aaaaaaaaaa cccccccccc cctctgacaa attgccctca gcacacactg        60

agggcaattt gtcacagg                                                      78


<210>  7
<211>  78
<212>  DNA
<213>  Artificial sequence

<220>
<223>  Disrupted RepE binding site

<400>  7
aaaaaaaaaa aaaaaaaaaa cccccccccc cctctgacaa attgccctca gcacacactg        60

agggcaattt gtcagagg                                                      78
```